(19) Europäisches Patentamt
European Patent Office
Office européen des brevets

(11) EP 2 797 958 B1

(12) **EUROPEAN PATENT SPECIFICATION**

(45) Date of publication and mention of the grant of the patent:
**19.07.2023 Bulletin 2023/29**

(21) Application number: **12863859.0**

(22) Date of filing: **27.12.2012**

(51) International Patent Classification (IPC):
***C07K 16/46*** *(2006.01)* ***A61K 39/395*** *(2006.01)*
***A61P 35/00*** *(2006.01)* *C07K 16/18 (2006.01)*
*C07K 16/28 (2006.01)* *C07K 16/30 (2006.01)*
*C07K 16/32 (2006.01)*

(52) Cooperative Patent Classification (CPC):
**A61P 35/00; C07K 16/2809; C07K 16/2887; C07K 16/30; C07K 16/32; C07K 16/468;**
A61K 2039/505; C07K 2317/52; C07K 2317/522; C07K 2317/53; C07K 2317/622; C07K 2317/64; C07K 2317/73; C07K 2317/92; C07K 2317/94;

(Cont.)

(86) International application number:
**PCT/US2012/071780**

(87) International publication number:
**WO 2013/101909 (04.07.2013 Gazette 2013/27)**

(54) **LIGHT CHAIN-BRIDGED BISPECIFIC ANTIBODY**

LEICHTKETTIGER ÜBERBRÜCKTER BISPEZIFISCHER ANTIKÖRPER

ANTICORPS BISPÉCIFIQUE À CHAÎNE LÉGÈRE PONTÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2011 US 201161580491 P**

(43) Date of publication of application:
**05.11.2014 Bulletin 2014/45**

(73) Proprietor: **Development Center for Biotechnology**
**New Taipei City 221 (TW)**

(72) Inventors:
- **HSU, Yu-shen**
  **New Taipei City 221 (TW)**
- **SHEU, Show-shan**
  **New Taipei City 221 (TW)**
- **CHANG, Ming-I**
  **New Taipei City 235 (TW)**
- **LO, Cheng-kai**
  **New Taipei City 221 (TW)**

(74) Representative: **Mewburn Ellis LLP**
**Aurora Building**
**Counterslip**
**Bristol BS1 6BX (GB)**

(56) References cited:
**WO-A1-94/04690** **WO-A1-95/09917**
**WO-A1-2011/047180** **WO-A2-2007/044887**
**WO-A2-2009/082624** **US-A1- 2011 076 268**

- **JOST C R ET AL: "A SINGLE-CHAIN BISPECIFIC FV2 MOLECULE PRODUCED IN MAMMALIAN CELLS REDIRECTS LYSIS BY ACTIVATED CTL", MOLECULAR IMMUNOLOGY, PERGAMON, GB, vol. 33, no. 2, 1 February 1996 (1996-02-01), pages 211-219, XP000646041, ISSN: 0161-5890, DOI: 10.1016/0161-5890(95)00087-9**
- **CHAMES PATRICK ET AL: "Bispecific antibodies for cancer therapy. The light at the end of the tunnel?", MABS, LANDES BIOSCIENCE, US, vol. 1, no. 6, 1 November 2009 (2009-11-01), pages 539-547, XP002688758, ISSN: 1942-0862**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 797 958 B1

- **KAZUHIKO KASUYA ET AL: "Bispecific anti-HER2 and CD16 single-chain antibody production prolongs the use of stem cell-like cell transplantation against HER2-overexpressing cancer", INTERNATIONAL JOURNAL OF MOLECULAR MEDICINE, SPANDIDOS PUBLICATIONS, GR , vol. 25, no. 2 1 February 2010 (2010-02-01), pages 209-215, XP002679897, ISSN: 1107-3756, DOI: 10.3892/IJMM_00000332 Retrieved from the Internet: URL:http://www.spandidos-publications.com/ijmm/25/2/209**
- **LU D ET AL: "Fab-scFv fusion protein: an efficient approach to production of bispecific antibody fragments", JOURNAL OF IMMUNOLOGICAL METH, ELSEVIER SCIENCE PUBLISHERS B.V.,AMSTERDAM, NL, vol. 267, no. 2, 15 September 2002 (2002-09-15), pages 213-226, XP004375842, ISSN: 0022-1759, DOI: 10.1016/S0022-1759(02)00148-5**


Remarks:

The complete document including Reference Table(s) and the Sequence Listing(s) can be downloaded from the EPO website

(52) Cooperative Patent Classification (CPC): (Cont.)
C07K 2319/00

## Description

### FIELD OF THE INVENTION

This invention relates to methods for preparing bispecific biomolecules, such as bispecific antibodies, and products thereof.

### BACKGROUND OF THE INVENTION

Combining biological molecules having different functions may lead to new molecules with desired or improved properties. For example, recent advances in multi-specific antibodies, including bispecific antibodies and bispecific tumor targeting T-lymphocyte engagers, have demonstrated that multi-specific molecules with immunological activating properties often deliver enhanced efficacies in tumor therapies, as compared to monoclonal antitumor therapies.

For example, a bispecific antibody (BsAb) may be composed of fragments of two different binding domains (i.e., variable regions) from different antibodies. The two different binding domains can bind two different types of antigens. These molecules may find applications in clinical therapies, such as cancer immunotherapy. For such applications, BsAbs may be designed to simultaneously bind a cytotoxic cell (using a receptor like CD3 (Kurby immunology. San Francisco:W.H. Freeman. ISBN 1-492-0211-4)) and a target such as a cancer cell (e.g., an antigen on the cancer cell).

Construction of bispecific or multi-specific proteins involves fusion of multiple protein domains. However, such fusions may cause incompatibility between fusion partners due to their differing biochemical and/or physiological properties. In addition, physiological limitations, including renal filtration or permeability of circulation pathways, may also challenge the designs of medically useable multi-specific tumor targeting molecules. Jost *et al* disclose a single chain bispecific $Fv_2$ molecule produced in mammalian cells, which redirects lysis by activated CTL (Mol. Imm. 33;2, pp211-219, 1996). Chames and Baty disclose bispecific antibodies for cancer therapy (Landes Bioscience, 1;6 pp529-547, 2009). Kasuya *et al* disclose a monomeric bispecific fusion protein comprising an anti-HER2 scFv and an anti-CD16 scFv which are connected to each other by a linker, a human kappa light chain constant domain of an immunoglobulin and a further linker (International Journal of Molecular Medicine, 25;2, pp209-215, 2010).

### SUMMARY OF THE INVENTION

Embodiments of the invention relate to novel formats of bispecific fusion proteins, defined by the claims, with immune activating properties for biomedical applications, such as clinical therapies. These proteins include two specific binding domains (targeting domains) connected by a bridging domain, which consists of a full-length kappa or lambda-2 light chain constant domain of an immunoglobulin, wherein the first targeting domain is directly linked with the bridging domain.

The bispecific fusion protein in accordance with embodiments of the invention may comprise an anti-tumor biomarker, such as CD20 (Hybridoma. 1983 2; 17), Her2/neu (Am J Clin Pathol 2003 120 (suppl 1); S53) or EpCAM (J. Immunol. 1992 148 (2); 590), and comprises a single chain linked Fv fragment (ScFv) as the cell-targeting or biomolecule-targeting domain, a kappa or lambda light chain constant domain of an immunoglobulin as a bridge, a linker , and an anti-CD3 ScFv as a T-lymphocyte activating domain. Such a light chain-bridged bispecific immune activator shows binding specificities for both tumor-expressed cellular targets and T-lymphocytes. Binding to both targets specifically induces T-lymphocyte-mediated cytotoxicity to tumor targets.

In addition to the anti-tumor ScFv examples described above, other cell-targeting domains may also be used. Such other molecules may have specific binding capabilities to other cellular targets. Examples of other cell-targeting domains, other than the T-lymphocyte activating domain, may include a toxin polypeptide, an enzyme, a hormone, a cytokine, a signaling molecule or ScFv of a desired specificity. Moreover, fusion proteins in accordance with embodiments of the invention may be expressed in prokaryotic or eukaryotic cells. In addition, the orientations of ScFv may be either light-chain variable regions linked to heavy-chain variable regions, or vise versa. The applications of such bispecific molecules include pharmaceutical applications, such as specific biomarker-bearing cell depletion therapies, such as cancer therapies. In addition, such molecules may be used in diagnostic applications.

One aspect of the invention relates to bispecific fusion proteins according to claim 1. A bispecific fusion protein in accordance with one embodiment of the invention may include a first targeting domain with a specificity for a first target of interest; a bridging domain consisting of a full length kappa or lambda-2 light chain constant domain of an immunoglobulin, which may be a human immunoglobulin; and a second targeting domain with a specificity for CD3. A targeting domain may be specific for a target biomolecule or a cell.

In accordance with the invention, a bispecific fusion protein further includes a linker fused to the C-terminus of the bridging domain. The first targeting domain is fused to the bridging domain and the second targeting domain is fused to the linker. The linker includes a GGGGS sequence. The first target of interest may be CD20, Her2/neu, EpCAM, or the like, and the second targeting domain is anti-CD3.

In any of the above embodiments, the first targeting domain may comprise a first ScFv with a specificity for the first target of interest, and the second targeting domain may comprise a second ScFv with a specificity for CD3.

In any of the above embodiments, each of the first ScFv and the second ScFv may comprise a human immunoglobulin sequence. In the above embodiments, the first ScFv may comprise VH-linker-VL or VL-linker-VH having a binding specificity for a first antigen and the second ScFv may comprise VH-linker-VL or VL-linker-VH having a binding specificity for CD3.

In any of the above embodiment, a linker may comprise one or more GGGGS (G4S) sequences. For example, the linker may comprise one G4S sequence, two G4S sequences (repeat), three G4S sequences, etc. In addition, the linker may comprise other amino acid sequences in combination with the G4S sequence. Such other amino acid sequences, for example, may include a hinge sequence (e.g., CPPCP).

Other aspects and advantages of the invention will be apparent from the following description and the appended claims.

## BRIEF DESCRIPTION OF THE DRAWINGS

FIG. 1A and FIG. 1B show schematics illustrating constructs of various bispecific T-lymphocyte activators.

FIG. 2A and FIG. 2B show results of expression and purification of light-chain bridged bispecific T-lymphocyte activator from eukaryotic and prokaryotic expression systems in accordance with embodiments of the invention. Figure 2B shows Coomassie brilliant blue stain (Panel A) and Western blotting (Panel B) against His-tagged LCBTAs on soluble proteins expressed by BL-21(DE3)pLysS. Lane 1 and 3 of both panels are extracts prior to IPTG induction. Lane 2 and 4 of both panels are extracts with IPTG induction. Lane 1 and 2 represent EpCAM targeting Ka LCBTA-1. Lane 3 and 4 represent Her2/neu targeting Ka LCBTA-1.

FIG. 3A shows Size Exclusion-High Performance Liquid Chromotpgraphy (SEC-HPLC) analysis of selected tumor targeting monoclonal antibody and light-chain bridged bispecific T-lymphocyte activators. FIG. 3B shows SEC-HPLC analysis of homogeneity of light chain-bridged bispecific T-lymphocyte activators and their antigen binding acitivities and improvement of yield.

FIGs.4A and 4B show examples of different tumor targeting light-chain bridged bispecific antibody binding to $CD20^+$ Raji lymphoma cells, Her2/$neu^+$ BT474 breast cancer cells, $EpCAM^+$ HT29 colorectal cancer cells (FIG. 4A), and CD3 of Jurkat lymphoma cells (FIG. 4B) in accordance with embodiments of the invention.

FIG. 5 shows comparison of transient expressions of Ig light chain-bridged bispecific T-lymphocyte activators of several bispecific antibodies in accordance with embodiments of the invention.

FIG. 6 demonstrates the in vitro serum stability analysis of La LCBTA and Ka LCBTA.

FIG. 7 shows examples of cytokine secretion profiles by peripheral blood mononuclear cells (PBMC) following stimulation of monoclonal antibody and tumor targeting bispecific antibodies (both EpCAM and Her2 targeting) in accordance with embodiments of the invention.

FIG. 8A shows cytotoxicities of Ig light chain-bridged bispecific T-lymphocyte activator to $CD20^+$ B-lymphoma (Raji) in accordance with embodiments of the invention. FIG. 8B shows cytotoxicities of Ig light chain-bridged bispecific T-lymphocyte activator to Her2/*neu*$^+$/$EpCAM^+$colorectal cancer (HT29) in accordance with embodiments of the invention. FIG. 8C shows cytotoxicities of Ig light chain-bridged bispecific T-lymphocyte activator to Her2/*neu*$^+$/Ep-$CAM^+$ pancreatic cancer(Capan-1) in accordance with embodiments of the invention.

FIG. 9A shows xenograft studies of tumor eradication by CD20 targeting Ig light chain-bridged bispecific T-lymphocyte activators in accordance with embodiments of the invention. FIG. 9B shows xenograft studies of tumor eradication by Her2/neu targeting Ig light chain-bridged bispecific T-lymphocyte activators in accordance with embodiments of the invention. FIG. 9C shows xenograft studies of tumor eradication by EpCAM targeting Ig light chain bridged bispecific T-lymphocyte activators in accordance with embodiments of the invention.

## DETAILED DESCRIPTION

Embodiments of the invention relate to novel formats of bispecific fusion proteins according to the claims, with immune-activating properties for biomedical applications, such as clinical therapies. Monomeric bispecific immune-activating molecules in accordance with embodiments of the invention may be referred to as light chain-bridged bispecific immune activators (LCBTA), or more generally as immunoglobulin-bridged bispecific biomolecules.

A bispecific molecule in accordance with embodiments of the invention comprises a bridging domain that links two targeting domains. A bridging domain in accordance with the invention comprises a full-length kappa or lambda-2 light chain constant domain of an immunoglobulin. The two targeting domains in accordance with embodiments of the invention may be derived from specific binding domains (i.e., variable regions) of antibodies. One of the two targeting domains may be a T-lymphocyte-activating domain, while the other targeting domain may be specific for a target molecule or cell, such as tumor cell, a tumor antigen, a virus, a bacterium, etc.

In accordance with embodiments of the invention, a targeting domain (TD) (e.g., a tumor-targeting domain, an antigen-targeting domain, a biomolecule-targeting domain, etc.) may be derived from the variable regions of an antibody. An antibody-derived targeting domain may comprise both a light-chain variable region and a heavy-chain variable region, which may be present in two configurations (orientations): (1) the light-chain variable region is at the N-terminus and the heavy-chain variable region is at the C-terminus, or (2) the heavy-chain variable region is at the N-terminus and the light-chain variable region is at the C-terminus.

In a targeting domain (e.g., a tumor-targeting domain), the heavy-chain variable region and the light-chain variable region may be connected with a linker, which may comprise any suitable linker, such as a short peptide fragment. For example, a linker in accordance with embodiments of the invention may comprise a short peptide, which typically comprises small amino acid residues or hydrophilic amino acid residues (e.g., glycine, serine, threonine, proline, aspartic acid, asparagine, etc.). One example of such a peptide is Gly-Gly-Gly-Gly-Ser (G4S). Other examples may include permutations of these amino acids in the sequence - such as GGGSG, GGSGG, GSGGG, or SGGGG. Further examples may include peptides containing amino acid residues other than G or S - such as GGTGS, GTSPGG, GNGGGS, etc. One skilled in the art would appreciate that many commonly used peptide linkers may be used in embodiments of the invention.

In accordance with some embodiments of the invention, such short peptide linkers may comprise repeat units to increase the linker length. For example, some linkers may comprise two G4S-repeated linkers, three G4S-repeated linkers, or four G4S-repeated linkers. Furthermore, some "repeat-like" linkers may comprise a mix of different peptide sequences - such as G4S - GGSGG - G4S - SGGGG.

In other disclosures, a bridging domain may comprise a peptide fragment, preferably a fragment derived from an antibody. In some disclosures, a bridging domain may be derived from a heavy chain, a light chain, particularly a constant region in a heavy or light chain. For example, a bridge in accordance with embodiments of the invention may be derived from a light chain, such as a kappa ($\kappa$) chain, a lambda-2 ($\lambda$-2) chain, or a lambda-5 ($\lambda$-5) chain (or a surrogate light chain). In some disclosures, a bridge may comprise a region or domain derived from a light chain, such as the constant region of a kappa ($\kappa$) chain, a lambda-2 ($\lambda$-2) chain, or a lambda-5 ($\lambda$-5) chain. Similarly, in some disclosures, a bridging domain may be derived from a heavy chain or a region (e.g., a constant region) of a heavy chain. As used herein, the term "derived from" refers to the fact a bridging domain shares a substantial homology (e.g., $\geq$ 50%, preferably $\geq$ 70%, more preferably $\geq$ 80%, most preferably $\geq$ 90%) with the full-length sequence of a domain (e.g., a constant region) of a light chain or a heavy chain.

In addition, in accordance with some disclosures, a bridging domain may be a mutant of the kappa chain, the Lambda chain, or the Lambda-5 (or a surrogate light chain) that mimics the light chain constant region, or a derivative of the kappa chain, the Lambda chain, or the Lambda-5 surrogate light chain. A "mutant" as used herein refers to a conserved mutant or a non-conserved mutant. A mutant in accordance with this disclosure retains the function of serving as a bridging domain, as described herein. One skilled in the art would appreciate that a conserved mutant comprises substitutions of amino acids with similar amino acids and typically would have preserved biological activities or structures. A conserved mutant may include 1-50 amino acid substitutions, preferably 1-30 amino acids, more preferably 1-20 amino acids, and most preferably 1-10 amino acids. A non-conserved mutant may have deletion, substitution, or insertion of one or more amino acid residues, for example 1-50 amino acids, preferably 1-30 amino acids, more preferably 1-20 amino acids, and most preferably 1-10 amino acids.

The linker between the bridging domain and the CD3 targeting domain may comprise a G4S linker or a G4S-repeat linker (which may be a double repeat, a triple repeat, or the like).

Various T-lymphocyte activating molecules are known in the art, including anti-CD3 antibodies (monoclonal or polycolonal), or the CD3-binding fragments of such antibodies or ligand or antibody to 4-1BB molecule. For example, in accordance with embodiments of the invention, a CD3 targeting domain may comprise the variable regions of a monoclonal antibody against CD3. In such embodiments, the heavy chain and the light chain of a variable region may be used in two orientations: (1) the light chain variable region at the N-terminus and the heavy chain variable region is at the C-terminus, or (2) the heavy chain variable region is at the N-terminus and the light chain variable region is at the C-terminus.

Similar to that described for the targeting domain, a linker may connect the heavy-chain variable region with the light-chain variable region of a T-lymphocyte binding domain. For example, such a linker may comprise any suitable amino acid sequences, such as a G4S or a double, triple, or quadruple G4S-repeated linker. In addition, the linkers may include other amino acid sequences.

Bispecific molecules of the invention can be used to target a cell or a molecule, while at the same time to activate T-lymphocyte responses. Some biological utilities of these molecules will be illustrated with the following working examples.

**Generation of immunoglobulin light chain-bridged bispecific T-lymphocyte activator**

In the first set of examples, an anti-CD20, anti-Her2/neu, or anti-EpCAM ScFv (single-chain fragment of variable regions) is used as a cell-targeting domain (CtD), and an anti-CD3 ScFv is chosen as a T-lymphocyte activating domain (TAD). The selected CtD and TAD are connected via a bridge, which for example may comprise an immunoglobulin domain derived from an antibody light chain or heavy chain. Examples of such bridges may include those selected from human immunoglobulin constant regions. Specific examples of such immunoglobulin chain bridges, selected from human immunoglobulin constant regions, may include, but not limited to, SEQ ID No. 1, SEQ ID No. 2, SEQ ID No. 3 and SEQ ID No. 4 shown below:

Sequence ID No. 1: V A A P S V F I F P P S D E Q L K S G T A S V V C L L N N F Y P R E A K V Q W K V D N A L Q S G N S Q E S V T E Q D S K D S T Y S L S S T L T L S K A D Y E K H K V Y A C E V T H Q G L S S P V T K S F N R G E C

- Human immunoglobulin kappa constant

•

Sequence ID No. 2: G Q P K A A P S V T L F P P S S E E L Q A N K A T L V C L I S D F Y P G A V T V A W K A D G S P V K A G V E T T T P S K Q S N N K Y A A S S Y L S L T P E Q W K S H R S Y S C Q V T H E G S T V E K T V A P T E C S

- Human immunoglobulin lambda 2 constant

•

Sequence ID No. 3: V T H V F G S G T Q L T V L S Q P K A T P S V T L F P P S S E E L Q A N K A T L V C L **Met** N D F Y P G I L T V T W K A D G T P I T Q G V E **Met** T T P S K Q S N N K Y A A S S Y L S L T P E Q W R S R R S Y S C Q V **Met** H E G S T V E K T V A P T E C S

- Human immunoglobulin lambda 5 constant

•

Sequence ID No. 4: T K G P S V F P L A P S S K S T S G G T A A L G C L V K D Y F P E P V T V S W N S G A L T S G V H T F P A V L Q S S G L Y S L S S V V T V P S S S L G T Q T Y I C N V N H K P S N T K V D K K A E P K S C

- Human immunoglobulin heavy chain constant 1 (CH1)

In accordance with the invention, the C-terminus of CtD is directly fused to the N-terminus of a kappa or lambda-2 light-chain bridge (e.g., a bridge derived from human immunoglobulin constant regions).

At the C-terminus of the light-chain bridge, a linker is fused with the C-terminus of the bridge. In accordance with the invention, said linker comprises one or more GGGGS sequences (G4S), with or without other sequences (e.g., a hinge sequence, CPPCP). This construct may then be linked to the TAD to form a bispecific molecule shown in FIG. 1.

Furthermore, one skilled in the art would appreciate that the CtD may be replaced with other specific binding domains for other target molecules.

The LCBTA shown in FIG. 1 is a bivalent (bispecific) molecule with one valence specific for a cellular target and the other specific for CD3 molecule on T-lymphocytes. This LCBTA can be expressed in mammalian, eukaryotic, or prokaryotic cells and purified, for example via light chain specific affinity columns, to homogeneity (FIG. 2). SEC-HPLC (size-exclusion HPLC) analysis showed that a great majority of the purified bispecific molecules are monomeric forms (FIG. 3). Nevertheless, the orientations of ScFv, the length of inter-domain linkers, compatibility of ScFv domains to bridging domains, and/or efficiency of translation may impact translation and post-translational processing of the bispecific molecules, which may result in elevated formation of multimers and/or altered biological functionality (I and J, FIG3, Table 1).

Table 1

| Tumor target | Bridge formats | Orientation of ScFvs | Linker formats | FACS analysis | | Homogeneity |
|---|---|---|---|---|---|---|
| | | | | CD3 binding % (10 $\mu$g/ml) | | |
| CD20 | kappa | HL HL | 3*G4S | 91.98 | | monomer, dimer |
| CD20 | kappa | HL LH | 3*G4S | 23.1 | | monomer |
| CD20 | lambda 2 | HL HL | 1xG4S | 88.8 | | monomer, dimer |
| CD20 | lambda 2 | LH LH | 1xG4S | 12.2 | | monomer |
| CD20 | kappa | HL LH | 3*G4S | 68.7 | | monomer, light dimer |
| EpCAM | kappa | HL LH | 3*G4S | 5.73 | | monomer |
| Her2/neu | kappa | LH HL | 3*G4S | 81.44 | | monomer, dimer |
| Her2/neu | kappa | HL LH | 3*G4S | 35.61 | | monomer, dimer |
| EpCAM | lambda 2 | HL LH | G4 S | 3.8 | | monomer, dimer |
| EpCAM | lambda 2 | LH HL | G4S | 74.46 | | monomer, dimer |
| | | | | EpCAM binding % | | |
| | | | | 10 $\mu$g/ml | 1 $\mu$g/ml | |
| EpCAM | kappa | HL LH | 3*G4S | 96.58 | 58.1 | monomer, light dimer |
| EpCAM | lambda 2 | HLLH | 3*G4S | 86.7 | 7.37 | monomer |

In addition to the effects of orientations, inter-domain linkers, compatibility between domains and translation and post translational modifications on monomer and multimer formations, the intra-domain linkers may also affect biological properties of the bispecific molecules. As shown in Figure 3B, for example, a Ka Her2/*neu* targeting LCBTA-1 comprises G4SG4SG4S intra-domain linker was engineered to comprise intra-domain linkers G4SG4S (Ka LCBTA-1-A) and G4SG4SG4SG4S(Ka LCBTA-1-B). Although SEC-HPLC and target binding analysis on three formats showed similar results, significant variations were found in the final protein yields.

**Construction of a reference bispecific antibody**

An IgG-FL (immunoglobulin G full length) bispecific antibody (BsAb) was constructed as a reference (see FIG. 1). This BsAb comprises a full-length anti-CD20 monoclonal antibody as a cell-targeting domain (CtD) at the N-terminus and two anti-CD3 ScFvs as a T-lymphocyte-activating domain (TAD) at the C-terminus. A linker/hinge domain comprising a GGGGSGGGGSCPPCPGGGGS peptide, which includes two linker (GGGGS or G4S) sequences and a hinge (CPPCP) sequence, maybe inserted between the CtD and the TAD of this reference IgG-FL BsAb (FIG. 1). This IgG-FL BsAb is a tetravalent molecule with two binding sites specific for cellular targets and the other two specific for CD3 molecules.

In addition to the above described IgG-FL BsAb, two tandem-repeat BsAbs were also constructed as references. As shown in FIG. 1, the tandem-repeat-1 has an anti-CD20 ScFv as the CtD at the N-terminus, a linker of GGGGS immediately following the C-terminus of the CtD, and an anti-CD3 ScFv as the TAD fused with the C-terminus of the linker. The tandem-repeat-2 has the CtD and the TAD in the reversed order of the tandem-repeat-1 - i.e., TAD at the N-terminus of the linker and CtD at the C-terminus of the linker.

**Binding assays**

The binding specificities for the cellular targets and T-lymphocyte are important part of the therapeutic indicators for bispecific molecules. The results for the above bispecific antibodies showed that LCBTA with mono-valent anti-CD20, anti-Her2/neu, or anti-EpCAM ScFv as the CtD could indeed bind Raji expressed CD20, BT474 expressed Her2/neu (ErbB2), or HT29 expressed EpCAM, respectively. The binding is close to a monoclonal anti-CD20, ant-Her2/neu or anti-EpCAM antibody (FIG. 4A). Fusion of the monovalent TAD (anti-CD3 ScFv) to the C-terminal of LCBTA reduced the binding affinity to Jurkat expressed CD3 molecules, as compared to a bivalent monoclonal anti-CD3 antibody (FIG. 4B). Such reduction is comparable to the bivalent reference format (IgG-FL BsAb) (FIG. 4B). The reference IgG-FL BsAb is a bispecific antibody resembling a full antibody, except that an anti-CD3 ScFv is covalently fused to the C-terminus of each heavy chain (FIG. 1).

**Expression of immunoglobulin light chain-bridged bispecific T-lymphocyte activator**

The expression of transiently transfected LCBTA in mammalian cell lines is illustrated in FIG. 2, 3, and 5. The reference formats (IgG-FL BsAb) showed expression rates no less than 1 μg/ml, which is similar to the lambda or kappa bridged LCBTA formats and their derivatives. The physiological properties of the bridged domains are important for the expression and stability of bispecific T-cell activator. For example, lambda 5, also known as surrogate light chain, is an immunoglobulin light-chain-like protein expressed by immature B-lymphocytes. The transient expression of lambda 5-bridged LCBTA by mammalian cells is reduced, as compared to Lambda or kappa bridged LCBTA. In addition, the CH1 of constant heavy chain domain from IgG1 as replacement for lambda or kappa bridge also resulted a poor rates of expression, as compared to the references, i.e., lambda or kappa bridged LCBTA.

The bridged-LCBTA composes 4 major domains; a tumor targeting domain, a light-chain bridge, a linker, and a T-cell activating domain. Alterations to any of theses domain may induce inter-domain interferences and result in reduced or loss of function, as compared with a full molecule. As shown in Table 1, changing the orientation/configuration of tumor targeting ScFv from light chain-heavy chain (Ka LCBTA-1) to heavy chain-light chain could result in reduced binding to T-cell activation domain, and vise versa. Such orientation manipulations also alter the formation of monomer production based on the SEC-HPLC analysis (FIG 3A). The linker domain in these examples comprises a short stretch of repetitive four glycines and a serine. The results showed that the lengths or sizes of inter-domain linkers can drastically affect the biological characteristic of bridged-LCBTA molecules (Table 2).

Table 2

| Tumor target | Bridge formats | Orientation of ScFvs | Linker formats | Cytotoxicity EC50 pM | Yield (transient mg/L) | Homogeneity |
|---|---|---|---|---|---|---|
| CD20 | kappa | LH HL | 3*G4S | 0.1 | 0.9 | monomer |
| CD20 | kappa | LH HL | 1*G4S | 10 | 0.4 | Monomer, dimer |

The bridged-LCBTAs expressed were also examined by SEC-HPLC to evaluate the contamination of multimeric LCBTAs. Elevated contamination of undesired multimeric bispecific molecules caused problems to other developers using ScFv as components. As shown in FIG. 3, regardless of the targeting molecules, a great majority of the purified bridged-LCBTAs are in monomeric forms. In addition, either kappa or lambda as bridged does not affect the homogeneity of monomers of LCBTAs.

The expressions of tandem repeat BsAbs are highly ScFv-dependent because reversing ScFv antibodies from N to C terminus, or vise versa could result in dramatic changes in the protein expression rates. As a result, the LCBTAs exhibited superior expression rates than the expression rates for the tandem repeat BsAb formats (see e.g., Lane 1 and 2, FIG. 5), and such difference was further amplified when TAD was place at the C-terminus of the molecules.

The short serum stability of ScFv or fragmented Ig molecule has been a common knowledge to Biopharmaceutical society (Cancer Res August 8, 2000 60; 433). With light chain as a bridge, the stabilities of ScFv-containing bispecific molecules are shown in FIG. 6. The results suggest both formats of LBCTA could stabilize over 50% of ScFv even after a week of 37°C incubation in serum.

**Inflammatory cytokine secretion profile by Ka LCBTA and monoclonal antibodies treated PBMC.**

Anti-CD3 monoclonal antibody treatment is known to induce activation of T-lymphocytes and enhanced secretion of inflammatory cytokines (FIG. 7). As shown in FIG. 7, other than IL-8, bridged-LCBTAs do not induce secretion of inflammatory cytokines. The bridged-LCBTAs do not comprise any heavy chain constant sequence, and, therefore, FcR mediated activation is not accountable for elevation of IL8. A low inflammatory cytokine secretions profile suggests reduced risks of side effects for T-lymphocyte dependent therapy.

**Cytotoxicity of immunoglobulin light chain-bridged bispecific T-lymphocyte activator**

To demonstrate the biological functionality of embodiments of the invention, CD20 targeted kappa-bridged LCBTA, anti-CD20 mAb and IgG-FL BsAbs were tested for their anti-tumor capabilities (FIGs. 1 and 8). As shown in FIG. 8, the kappa-bridged LCBTA is highly effective in tumor eradication with an EC50 value in the sub pM range, as compared to 22 pM for the anti-CD20 mAb or single digit pM for the IgG-FL BsAb. The kappa-bridged LCBTA also showed a maximal tumor eradication rate up to 90 %, while only 35% for anti-CD20 mAb, and 75% for IgG-FL BsAb.

To demonstrate the biological functionality of embodiments of the invention, either *Her2*/*neu* or EpCAM targeted kappa-bridged LCBTA were tested on Her2/*neu*$^+$ and EpCAM$^+$ HT29 colorectal carcinoma cells to evaluate their tumor eradication capabilities (FIG 8B). As shown in FIG. 8B, both *Her2*/*neu* and EpCAM targeting kappa-bridged LCBTA are highly effective in tumor eradication with a maximal cytotoxicity rate up to 100%, as compared to 28% to 51% tumor killing rates for the anti-Her2/neu and anti-EpCAM mAbs, respectively.

To demonstrate the biological functionalities of embodiments of the invention, either *Her2*/*neu* or EpCAM targeted kappa-bridged LCBTA were tested on Her2/*neu*$^+$ and EpCAM$^+$ Capan-1 pancreatic cancer cells to evaluate their tumor eradication capabilities (FIG 8C). As shown in FIG. 8C, both *Her2*/*neu* and EpCAM targeting kappa-bridged LCBTA are highly effective in tumor eradication with a maximal cytotoxicity rate over 75% and 100% for Her2/neu and EpCAM targeting kappa-bridged LCBTA, respectively, as compared to 39% to 34% tumor killing rates for the anti-Her2/neu and anti-EpCAM mAbs, respectively.

**Xenograft analysis on tumor bearing animals**

To demonstrate the therapeutic potentials of embodiments of the invention, CD20 targeted kappa-bridged LCBTA and anti-CD20 mAb were tested on CD20$^+$ human lymphoma (Raji cell)-bearing SCID mice for anti-tumor capabilities (FIGs. 1 and 8). Before the therapy, SCID mice were inoculated with Raji cells and PBMC, subcutaneously. As shown in FIG. 9A, animals treated with Ka LCBTA as a therapeutic agent developed much smaller tumors than animals treated with anti-CD20 mAb as a therapeutic agent.

To demonstrate the therapeutic potentials of embodiments of the invention, *Her2*/*neu* targeted kappa-bridged LCBTA and anti-Her2/*neu* mAb were tested on Her2/*neu*$^+$ human colorectal carcinoma (HT29 cells)-bearing SCID mice for anti-tumor capabilities (FIGs. 1 and 8). Prior to the therapy, SCID mice were inoculated with HT29 cells pre-mixed with either preactiavted or naive PBMCs, subcutaneously. As shown in FIG. 9B, animals treated with Her2/*neu* targeting Ka LCBTA as a therapeutic agent developed significantly smaller tumor than animals treated with anti-Her2/*neu* mAb as a therapeutic agent.

To demonstrate the therapeutic potentials of embodiments of the invention, EpCAM targeted kappa-bridged LCBTA and anti-EpCAM mAb were tested on EpCAM$^+$ human colorectal carcinoma (HT29 cells)-bearing SCID mice for anti-tumor capabilities (FIGs. 1 and 8). Prior to the therapy, SCID mice were inoculated with HT29 cell pre-mixed with preactiavted PBMCs, subcutaneously. As shown in FIG. 9B, EpCAM targeting Ka LCBTA effectively inhibited tumor progression.

**Constructing Bispecific antibodies**

Restriction enzymes were purchased from various venders. DNA polymerase, T4 DNA ligase Klenow enzyme and T4 DNA polymerase were from Invitrogen (Grand Island, NY). All enzymes were used as recommended by the manufactures.

All primers for PCR amplifications were purchased from venders. DNA amplifications were performed in a PCR machine using a pre-denaturing step of 2 minutes at 94°C, followed by 35 cycles, containing a denaturing step (94°C), an annealing step (50°C), and an extension step (72°C), each for 50 seconds.

All expression modules are schematically represented in FIG. 1.

The anti-CD20, anti-Her2/neu and anti-EpCAM light chains and truncated heavy chains were cloned into vector vectors pGEM, separately. A single-chain fragment of anti-CD20, anti-Her2/neu and anti-EpCAM VH and VL was cloned into vector TCAE8 and used for subsequent anti-tumor ScFv.

**Cell Lines preparation**

The Raji, BT474, Capan-1 and HT29 cells used in this invention are B-lymphoma tumor cell line, breast cancer cell line, pancreatic cell line, and colorectal cancer cells line, respectively, obtained from Bioresource Collection and Research Center (BCRC), which is a division of Food Industry Research and Development Institute (FIRDI) in Taiwan, R.O.C. The Jurkat cell is a T-lymphoma cell line from ATCC. Both Raji and Jurkat cells are cultured in RPMI 1640 medium (GibcoBRL Life Technologies, Paisly, UK) supplemented with 10% Fetal bovine serum (Hyclone), 0.03% L-glutamine and 0.4 mM of sodium pyruvate. After incubation at 37°C humidified incubator containing 5% of $CO_2$, cells were subcultured or washed in sterilized buffer for testing. BT474, Capan-1 and HT29 were cultured according to the guidelines from ATCC.

**Preparation of peripheral blood mononuclear cells (PBMC)**

Peripheral blood mononuclear cells (PBMC) were isolated from whole blood of normal healthy adult donors with Ficoll-Paque PLUS by density centrifugation. Following the isolation, PBMC were cultured and pre-activated for 5-10 days in RPMI-1640 medium supplemented with 10 ng/ml of anti-CD3 mAb, 75 IU/ml of interlekine-2 (IL-2), and 10% FBS.

**Cytotoxicity Assays (Calcein AM cytotoxicity)**

The target cells (Raji) were labeled with 10 μM of Calcein for 30 min at 37°C in phenol red-free RPMI 1640 medium supplemented with 5% FBS. At the end of Calcein incubation, cells were washed twice with phenol red-free RPMI 1640 medium containing 5% FBS, and the cell density was adjusted to $3\times10^5$ cells/ml with phenol red-free RPMI 1640 containing 5% FBS. For the reaction mixture, 100 μl aliquots of medium each containing $3\times10^4$ cells were placed in each well of a 96-well culture plate. The cell density of effecter cells (PBMC) culture was calculated and adjusted to $3\times10^6$ cells/ml by phenol red-free RPMI 1640 medium containing 5% of FBS. For cytotoxic assays, different quantities of different BsAbs and 100 μl ($3\times10^5$ cells) of effecter cells were added into Raji preloaded, 96 well culture plate and incubated in a 37°C, 5% $CO_2$ enriched incubator for 4 hours. At the end of the incubation, the culture plate was centrifuged at 700 g for 5 minutes. Then, 130 μl of the supernatant from each reaction well was transferred, individually, to a new plate and the dye released was quantitated in Fusion alpha micro-plate reader. The percent of cytotoxicity was calculated according to the formula:

$$[\text{fluorescence (sample) - fluorescence (control)}]/[\text{fluorescence (total-lysis) - fluorescence (control)}]*100.$$

The total-lysis was defined as target cells treated with 0.9% of Triton for 10 minutes.

**Flow Cytometry Assays**

**Biding Affinity to Tumor Target (B-lymphoma)**

Target cells, including Raji, BT474 and HT29 cells ($1\times10^6$ cells/reaction) were treated with different BsAbs at different concentrations at room temperature for 30 minutes. At the end of the incubation, all reactions were washed twice with PBS supplemented with 2% of FBS. After wash, cells were re-incubated with 1 μl of FITC conjugated, affinity purified F(ab')2 fragment, goat anti-human IgG (Fab')2 fragment-specific antibody for 30 minutes at room temperature. Following the incubation, cells were washed twice with ice cold PBS supplemented with 2% FBS and monitored by FACS apparatus.

Jurkat cells ($1\times10^6$ cells/reaction) were treated with different BsAbs at different concentrations at room temperature for 30 minutes. At the end of the incubation, all reactions were washed twice with PBS supplemented with 2% of FBS. After wash, cells were re-incubated with 1 μl of FITC conjugated, affinity purified F(ab')2 fragment, goat anti-human IgG (Fab')2 fragment-specific antibody for 30 minutes at room temperature. Following the incubation, cells were washed twice with ice cold PBS supplemented with 2% FBS and monitored by FACS apparatus.

**Prokaryotic cloning and expression of Immunoglobulin light chain bridged bispecific antibodies**

Synthetic genes corresponding to the desirable tumor targeting light chain bridged bispecific antibodies were cloned into pET20b vector using Nco I and xhoI restriction sites. About 20 ng of each recombinant construct were transformed into Novagen® BL21(DE3)pLysS (EMD Millipore), and transformants were selected on LB agar plate supplemented with Ampicillin.(100 μg/ml ).

Single colonies of BL21(DE3)pLysS containing the recombinant constructs were grown overnight at 37 °C in 5 mL of LB supplemented with Ampicillin. Cultures were incubated at 37 °C under shaking conditions. The inducer (IPTG) was then added when populations reached an $OD_{600}$ of 0.6-0.7. Aliquots were harvested by centrifugation (4000×g for 15 min) after induction for 0 hr. and overnight. To verify the expression of target-specific light chain bridged bispecific antibodies, total protein extracts of soluble fraction were analyzed by SDS-PAGE (4-12% acrylamide). Samples were prepared by adding a sample buffer and boiled for 5 min. After separation, gels were stained with 0.1% Coomassie brilliant blue R-250. Western blot were analyzed using anti-His mAb.

The above examples demonstrate the utility of embodiments of the invention, which are bispecific molecules.

The above examples also illustrate that various targeting domains can be used to target different molecules or cells. Therefore, one skilled in the art would appreciate that other targeting domains for different targets may be used in the same framework of bispecific molecules described herein. Accordingly, the scope of the invention is not limited to the specific preferred embodiments shown in the examples.

While the invention has been described with respect to a limited number of embodiments, those skilled in the art, having benefit of this disclosure, will appreciate that other embodiments can be devised which do not depart from the scope of the invention as disclosed herein. Accordingly, the scope of the invention should be limited only by the attached claims.

**Claims**

1. A monomeric bispecific fusion protein consisting of a single polypeptide that comprises:

   a first targeting domain with a specificity for a first target of interest;
   a bridging domain consisting of a full-length kappa or lambda-2 light chain constant domain of an immunoglobulin;
   a second targeting domain with a specificity for CD3; and
   an inter-domain linker comprising a GGGGS sequence fused to the C-terminus of the bridging domain and to the second targeting domain,

   wherein the first targeting domain is directly linked with the bridging domain.

2. The monomeric bispecific fusion protein of claim 1, wherein the immunoglobulin is a human immunoglobulin.

3. The monomeric bispecific fusion protein of claim 1 or 2, wherein the first target of interest is CD20, Her2/neu, or EpCAM.

4. The monomeric bispecific fusion protein of any one of claims 1-3, wherein the first targeting domain comprises a first ScFv with a specificity for the first target of interest, and the second targeting domain comprises a second ScFv with a specificity for CD3.

5. The monomeric bispecific fusion protein of claim 4, wherein each of the first ScFv and the second ScFv comprises a human sequence.

6. The monomeric bispecific fusion protein of claim 4 or 5, wherein the first ScFv comprises VH-linker-VL or VL-linker-VH having a binding specificity for a first antigen and the second ScFv comprises VH-linker-VL or VL-linker-VH having a binding specificity for CD3.

7. The monomeric bispecific fusion protein of claim 6, wherein the ScFv linker comprises a GGGGS sequence.

**Patentansprüche**

1. Monomeres bispezifisches Fusionsprotein, bestehend aus einem einzelnen Polypeptid, das Folgendes umfasst:

   eine erste abzielende Domäne mit einer Spezifität für ein erstes Ziel von Interesse;
   eine Brückendomäne, die aus einer konstanten kappa- oder lambda-2-Leichtketten-domäne voller Länge eines Immunglobulins besteht;
   eine zweite abzielende Domäne mit einer Spezifität für CD3; und
   einen Zwischendomänenlinker, umfassend eine Sequenz GGGGS, die an den C-Terminus der Brückendomäne und an die zweite abzielende Domäne fusioniert ist,
   wobei die erste abzielende Domäne direkt an die Brückendomäne gebunden ist.

2. Monomeres bispezifisches Fusionsprotein nach Anspruch 1, wobei das Immunglobulin ein menschliches Immunglobulin ist.

3. Monomeres bispezifisches Fusionsprotein nach Anspruch 1 oder 2, wobei das erste Ziel von Interesse CD20, Her2/neu oder EpCAM ist.

4. Monomeres bispezifisches Fusionsprotein nach einem der Ansprüche 1 bis 3, wobei die erste abzielende Domäne ein erstes ScFv mit einer Spezifität für das erste Ziel von Interesse umfasst und die zweite abzielende Domäne ein zweites ScFv mit einer Spezifität für CD3 umfasst.

5. Monomeres bispezifisches Fusionsprotein nach Anspruch 4, wobei jedes aus dem ersten ScFv und dem zweiten ScFv eine menschliche Sequenz umfasst.

6. Monomeres bispezifisches Fusionsprotein nach Anspruch 4 oder 5, wobei das erste ScFv VH-Linker-VL oder VL-Linker-VH mit einer Bindungsspezifität für ein erstes Antigen umfasst und das zweite ScFv VH-Linker-VL oder VL-Linker-VH mit einer Bindungsspezifität für CD3 umfasst.

7. Monomeres bispezifisches Funktionsprotein nach Anspruch 6, wobei der ScFv-Linker eine Sequenz GGGGS umfasst.

## Revendications

1. Protéine de fusion bispécifique monomérique constituée d'un polypeptide unique qui comprend :

   un premier domaine de ciblage avec une spécificité pour une première cible d'intérêt ;
   un domaine de pontage constitué d'un domaine constant d'une chaîne légère kappa ou lambda-2 pleine longueur d'une immunoglobuline ;
   un second domaine de ciblage avec une spécificité pour CD3 ; et
   un lieur inter-domaine comprenant une séquence GGGGS fusionnée à l'extrémité C-terminale du domaine de pontage et au second domaine de ciblage, dans laquelle le premier domaine de ciblage est directement lié au domaine de pontage.

2. Protéine de fusion bispécifique monomérique selon la revendication 1, dans laquelle l'immunoglobuline est une immunoglobuline humaine.

3. Protéine de fusion bispécifique monomérique selon la revendication 1 ou 2, dans laquelle la première cible d'intérêt est CD20, Her2/neu ou EpCAM.

4. Procédé selon l'une quelconque des revendications 1 à 3, dans lequel le premier domaine de ciblage comprend un premier ScFv, fragment variable à chaîne unique, avec une spécificité pour la première cible d'intérêt, et le second domaine de ciblage comprend un second ScFv avec une spécificité pour CD3.

5. Protéine de fusion bispécifique monomérique selon la revendication 4, dans laquelle chacun du premier ScFv et du second ScFv comprend une séquence humaine.

6. Protéine de fusion bispécifique monomérique selon la revendication 4 ou 5, dans laquelle le premier ScFv comprend VH-lieur-VL ou VL-lieur-VH présentant une spécificité de liaison pour un premier antigène et le second ScFv comprend VH-lieur-VL ou VL-lieur-VH présentant une spécificité de liaison pour CD3.

7. Protéine de fusion bispécifique monomérique selon la revendication 6, dans laquelle le lieur de ScFv comprend une séquence GGG.

Figure 1 A

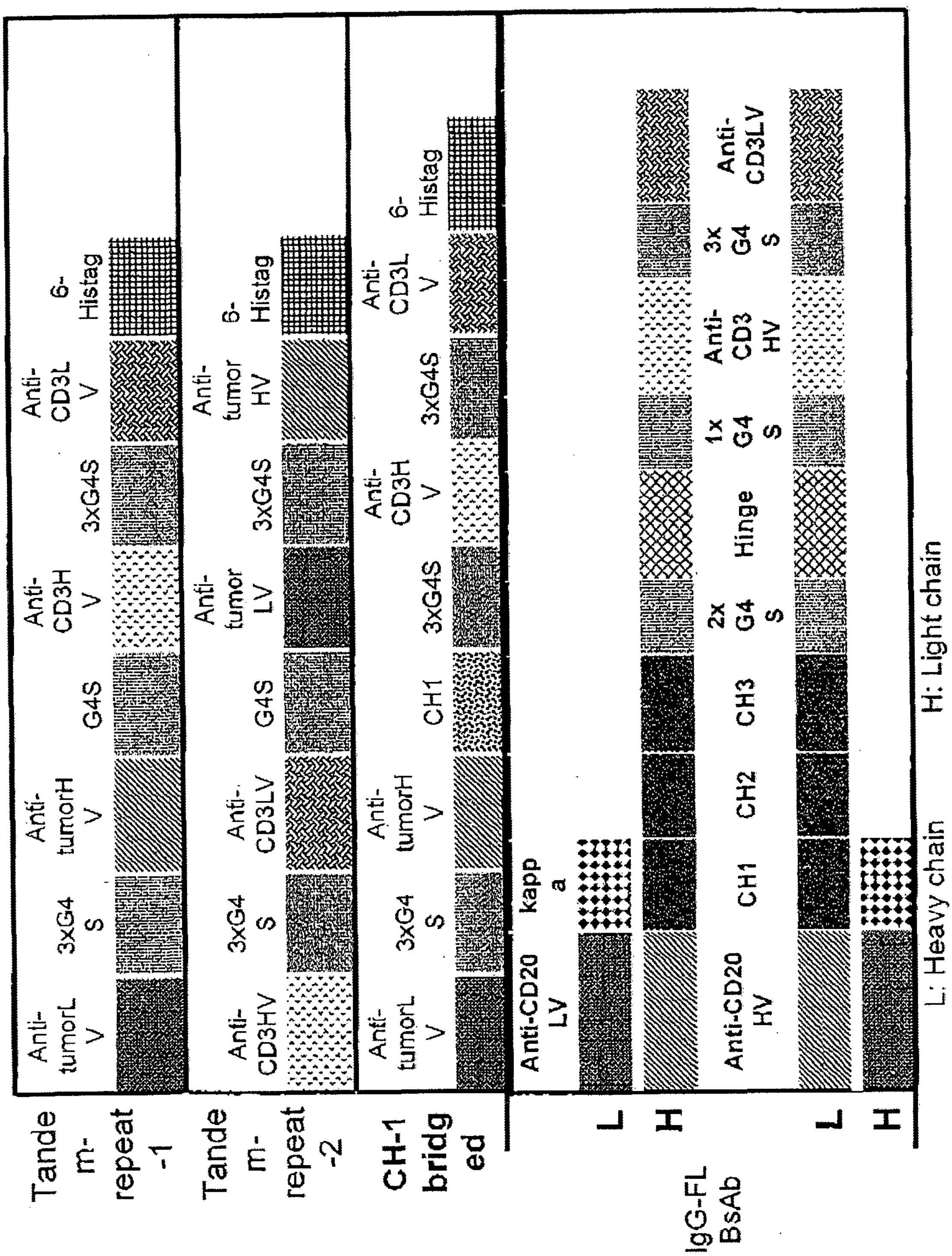
Tandem-repeat-1
Anti-tumorLV
3xG4S
Anti-tumorHV
G4S
Anti-CD3HV
3xG4S
Anti-CD3LV
6-Histag
Tandem-repeat-2
Anti-CD3HV
3xG4S
Anti-CD3LV
G4S
Anti-tumor LV
3xG4S
Anti-tumor HV
6-Histag
CH-1 bridged
Anti-tumorLV
3xG4S
Anti-tumorHV
CH1
3xG4S
Anti-CD3HV
3xG4S
Anti-CD3LV
6-Histag
IgG-FL BsAb
Anti-CD20 LV
kappa
L
H
Anti-CD20 HV
CH1
CH2
CH3
2x G4 S
Hinge
1x G4 S
Anti-CD3 HV
3x G4 S
Anti-CD3LV
L
H
L: Heavy chain
H: Light chain

Figure 1 A (Continued)

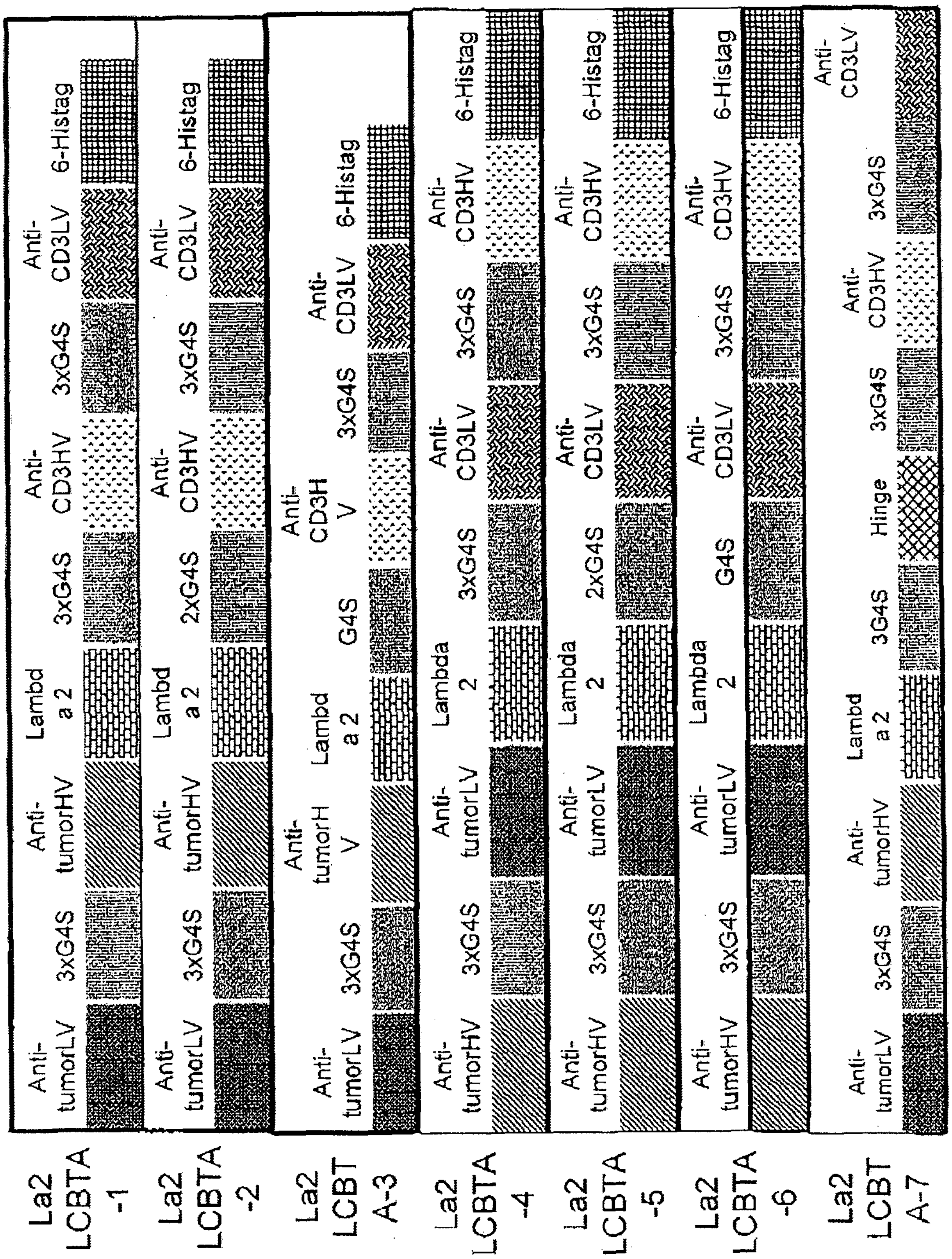

Figure 1 A (Continued)

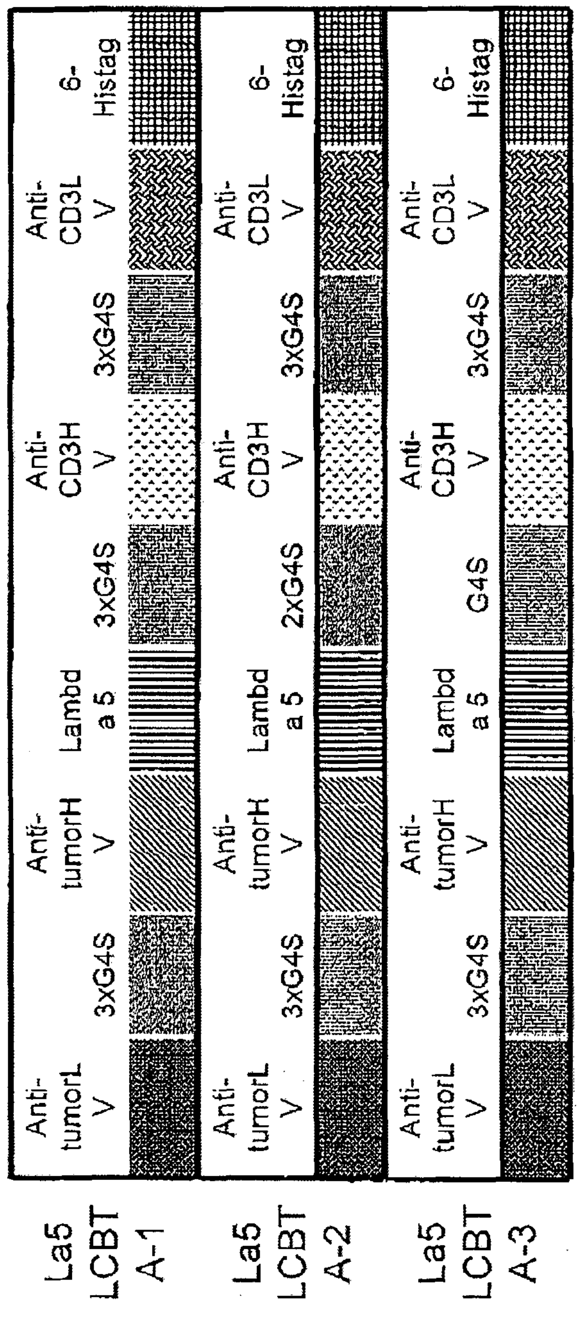

Figure 1 A (Continued)

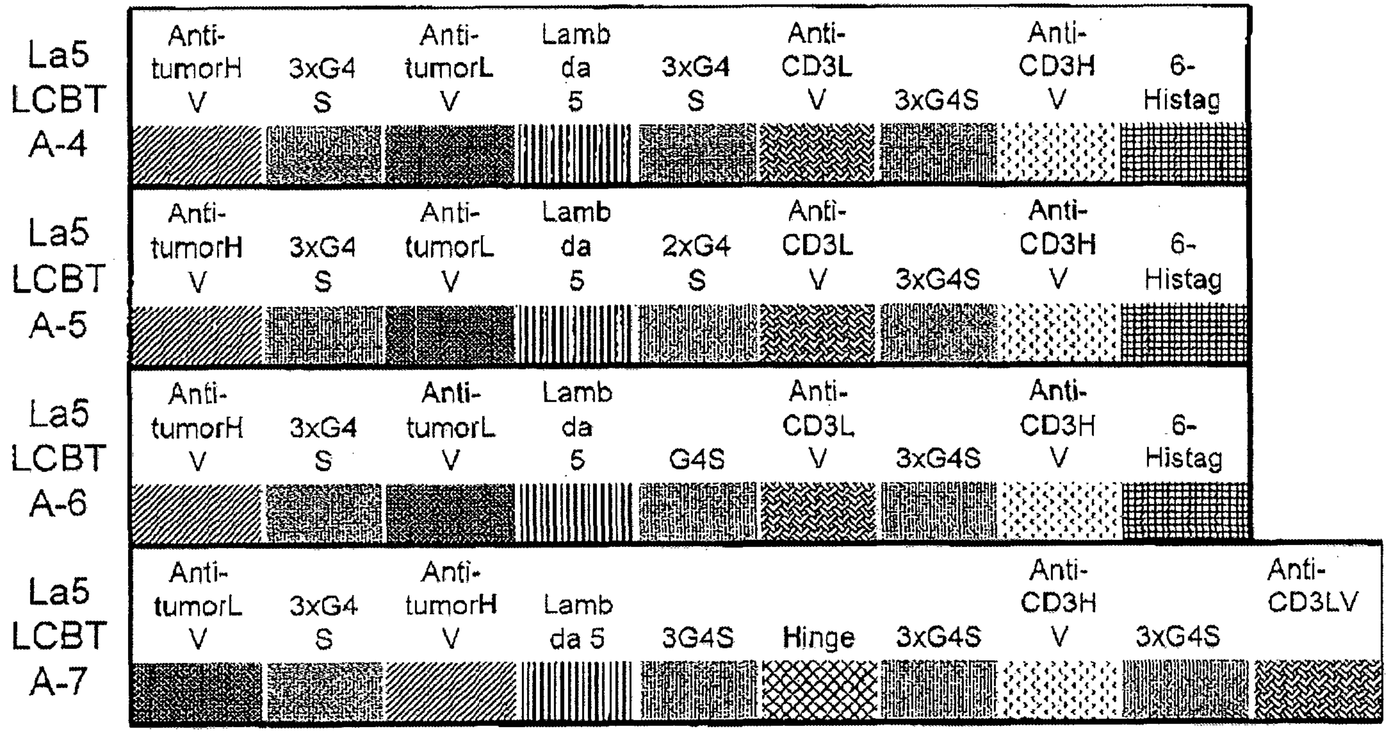

Figure 1 A (Continued)

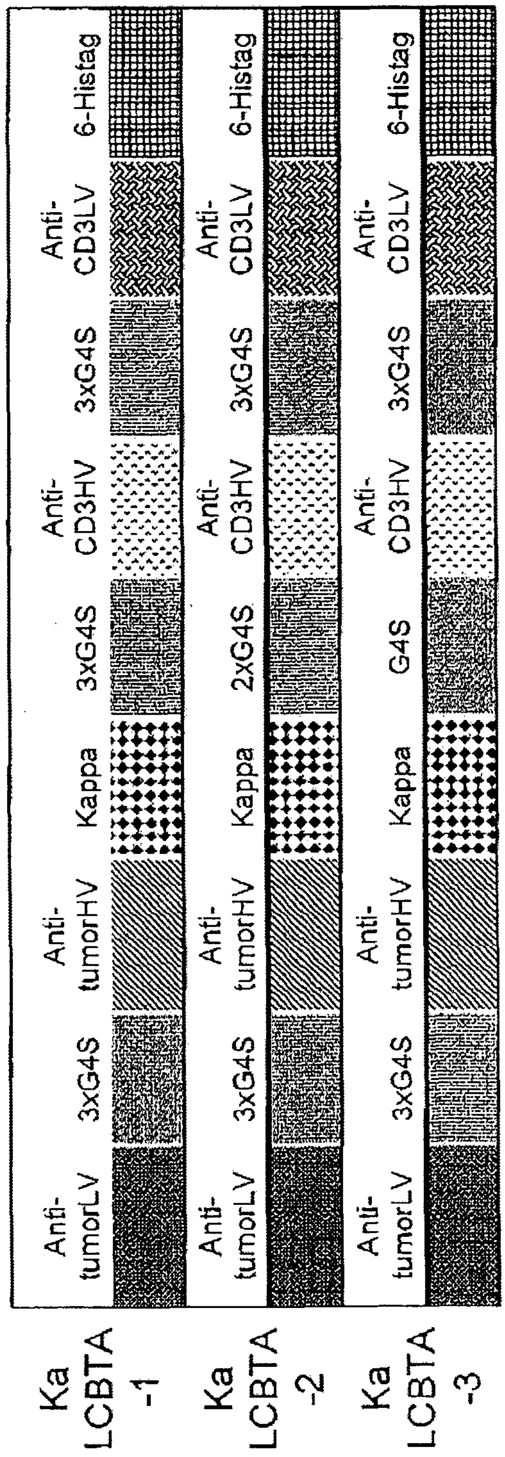

## Figure 1 A (Continued)

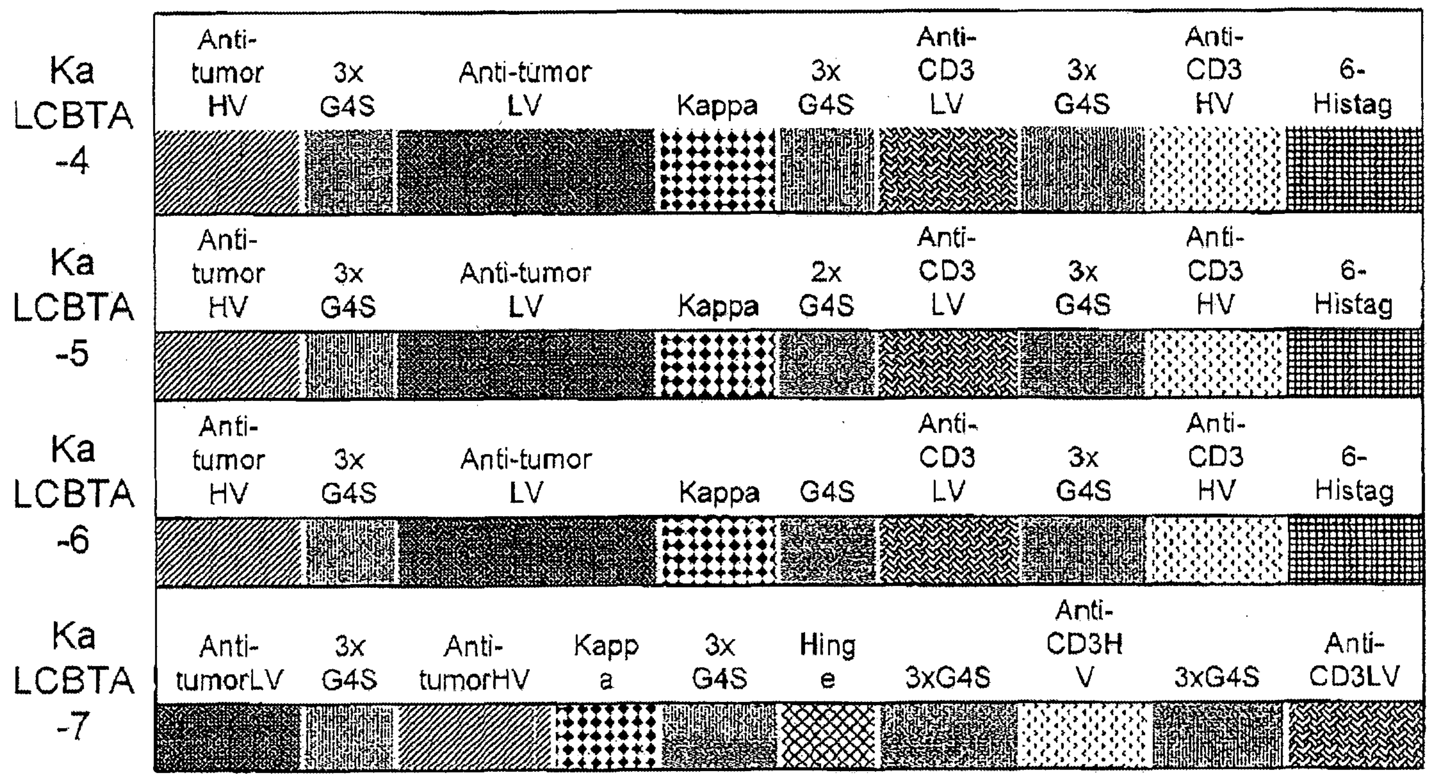

Figure 1 A (Continued)

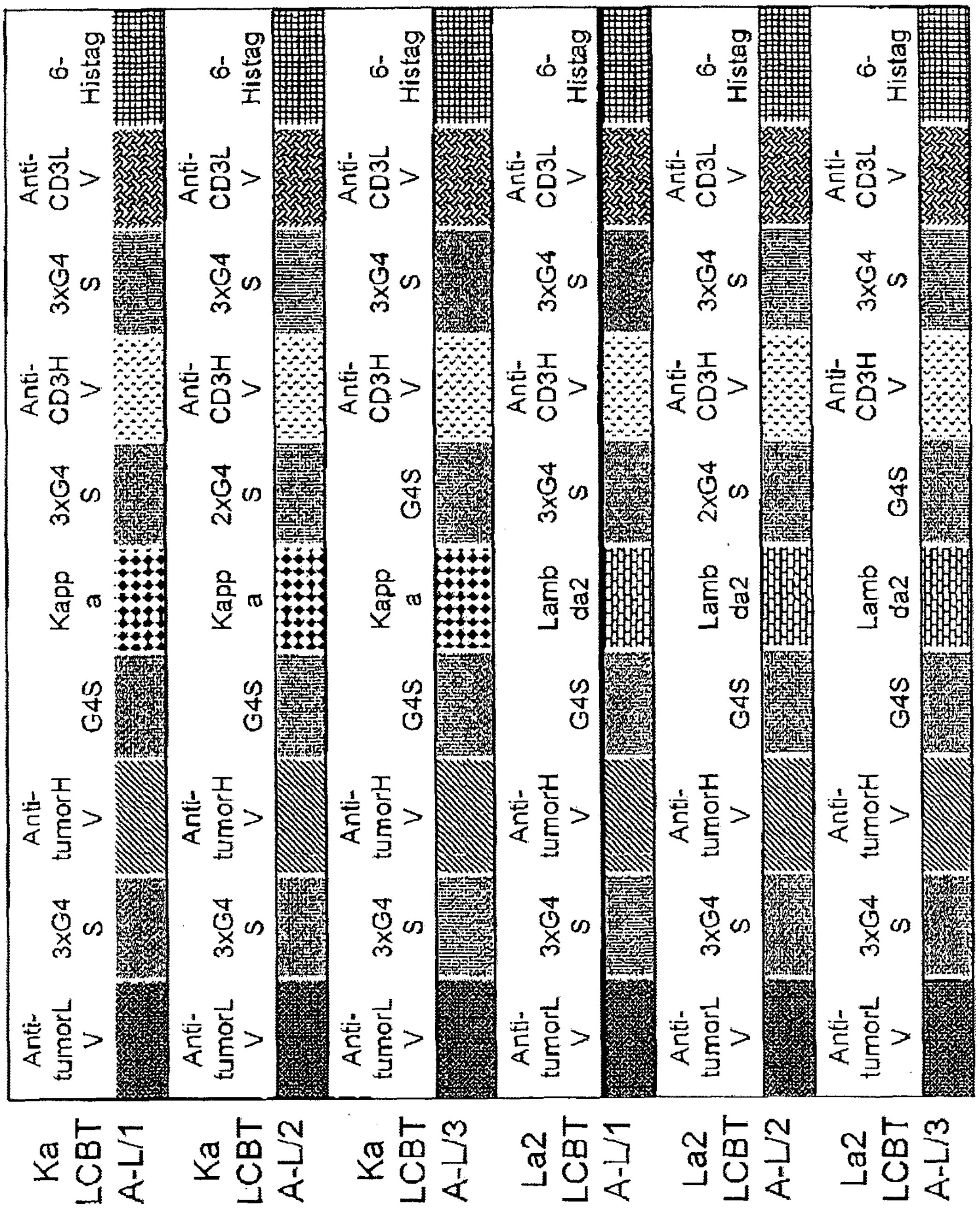

Figure 1 A (Continued)

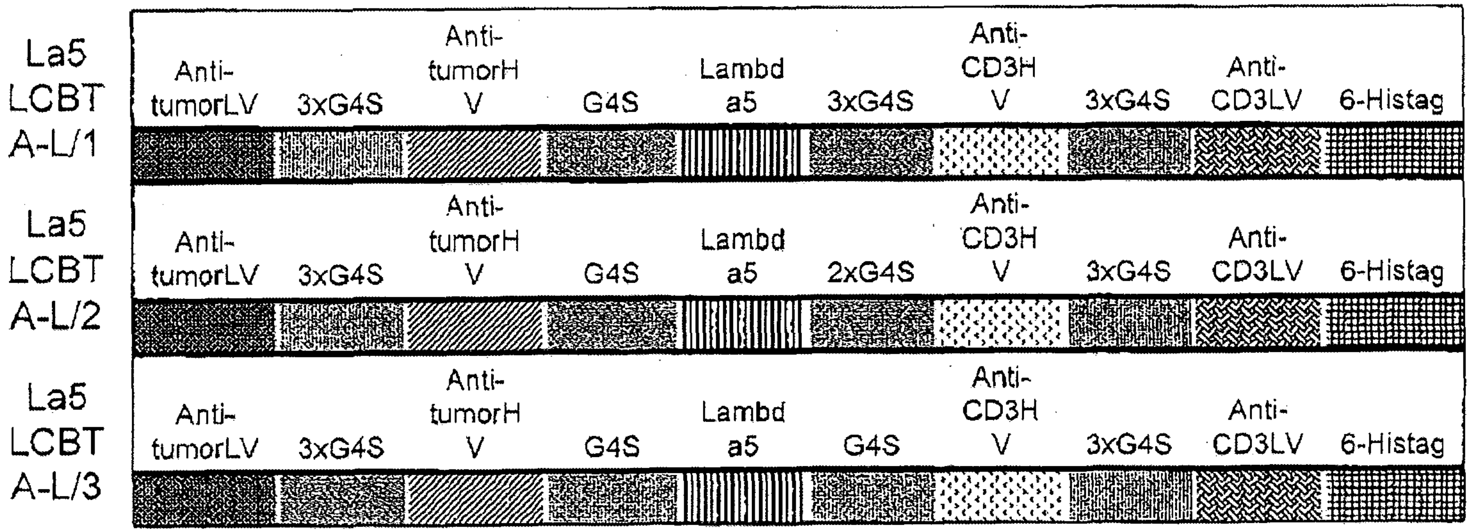


LV: light chain variable sequence
HV: heavy chain variable sequence
Anti-tumor: Anti-tumor specific marker ScFv
Anti-CD3: anti-human CD3 specific ScFv
G4S: glycine-glycine-glycine-glycine-serine (GGGGS) or linker sequence
3xG4S: three repeats of GGGGS linker sequence
6XHistag: Histadin-Histadin-Histadin-Histadin-Histadin-Histadin
Tumor markers: CD20, Her2/neu and EpCAM

Figure 1 B

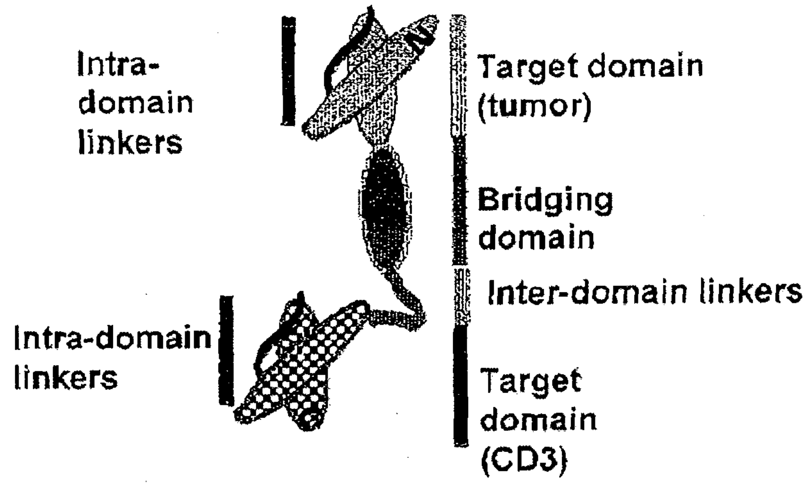


Intra-domain linker, for example, may comprise one of the following linker sequences:

1. GGGGSGGGGS
2. GGGGSGGGGSGGGGS
3. GGGGSGGGGSGGGGSGGGGS

Orientation of ScFv targeting domain comprises one of the following domain orientations:

1. Light chain variable-Heavy chain variable
2. Heavy chain variable-Light chain variable

Bridging domain, for example, may comprise one of the following light chain constant domains:

1. Kappa chain
2. Lambda 2 chain
3. Lambda 5 chain/a.k.a. Surrogate light chain

Inter-domain linker, for example, may comprise one of the following linker sequence

1. GGGGS
2. GGGGSGGGGS
3. GGGGSGGGGSGGGGS

Figure 2A

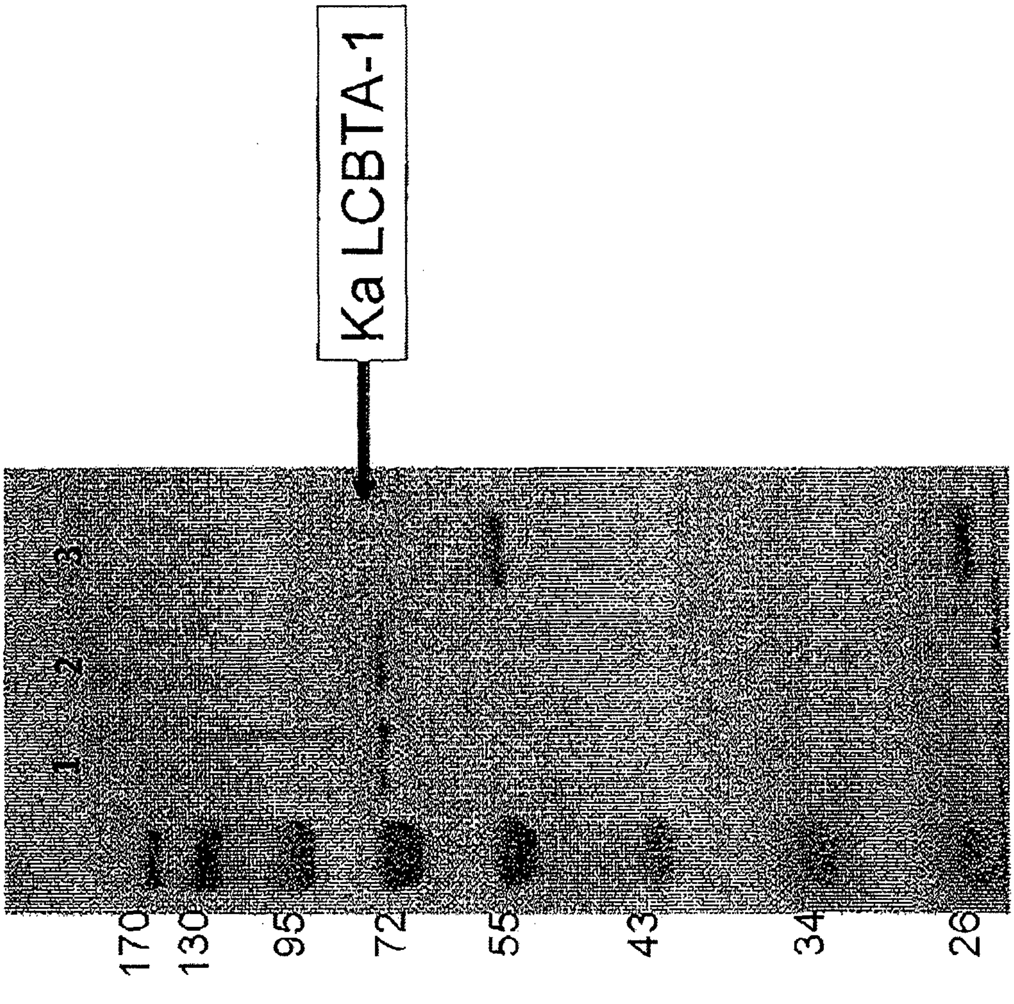

Figure 2B

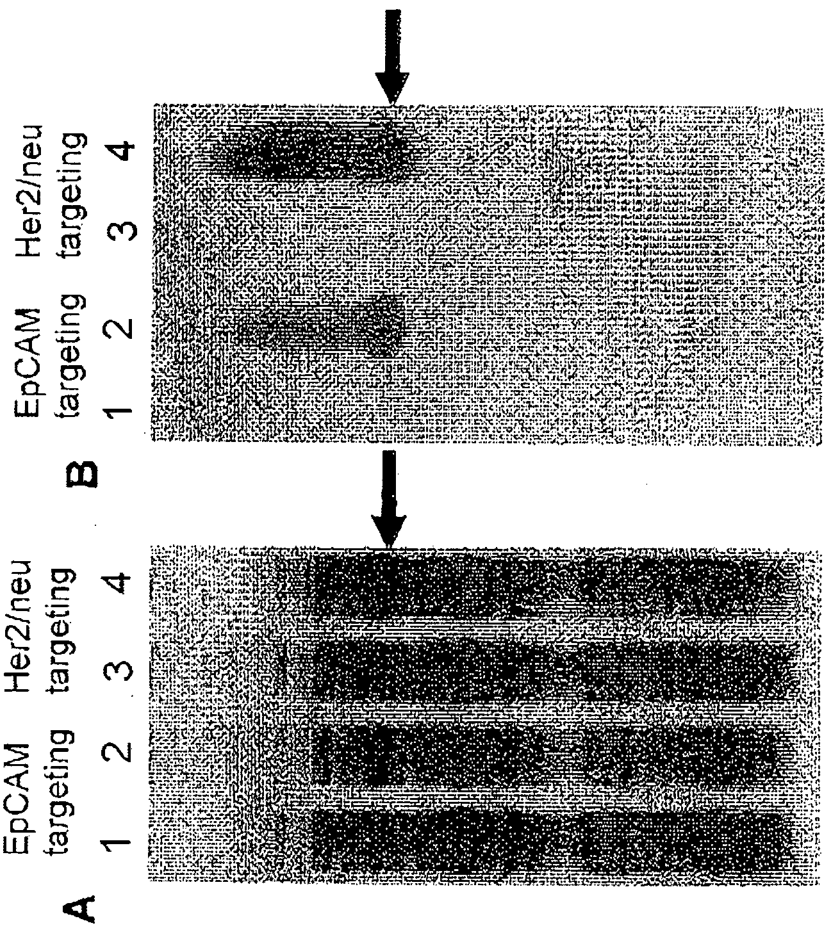
A
EpCAM targeting
1 2
Her2/neu targeting
3 4
B
EpCAM targeting
1 2
Her2/neu targeting
3 4

Figure 3A

A

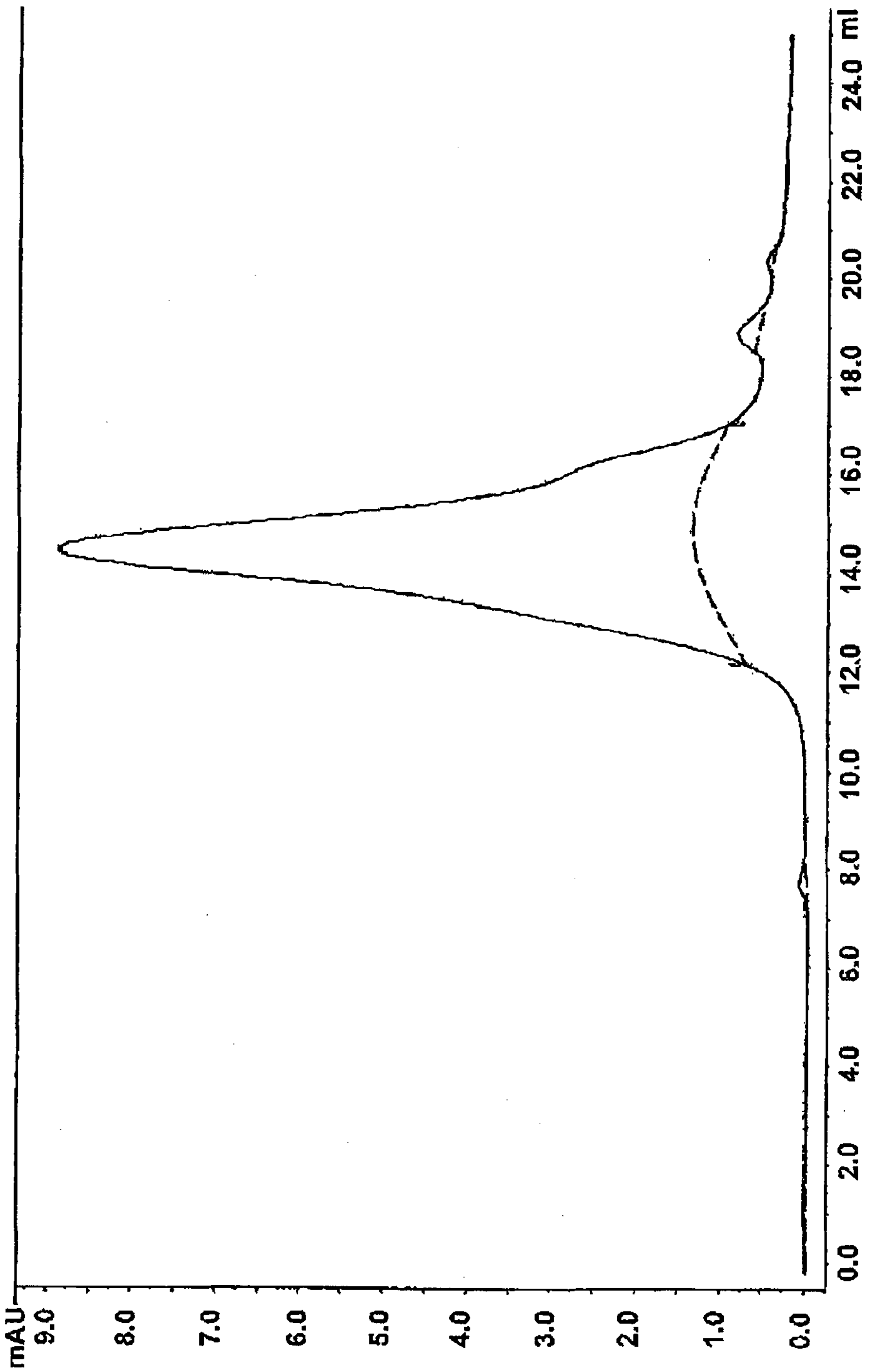
mAU
9.0
8.0
7.0
6.0
5.0
4.0
3.0
2.0
1.0
0.0
0.0
2.0
4.0
6.0
8.0
10.0
12.0
14.0
16.0
18.0
20.0
22.0
24.0
ml

Figure 3A

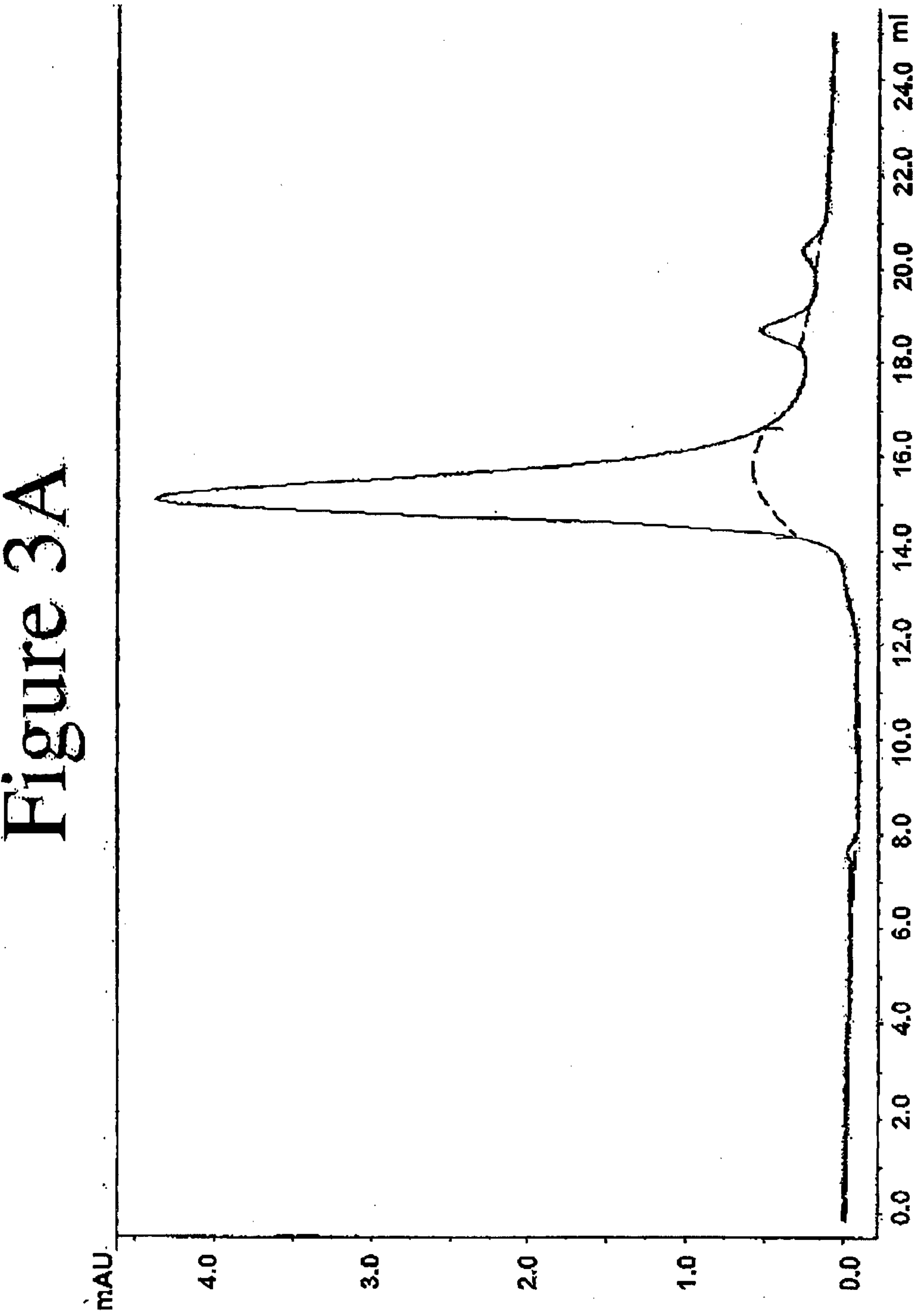


B

Figure 3A

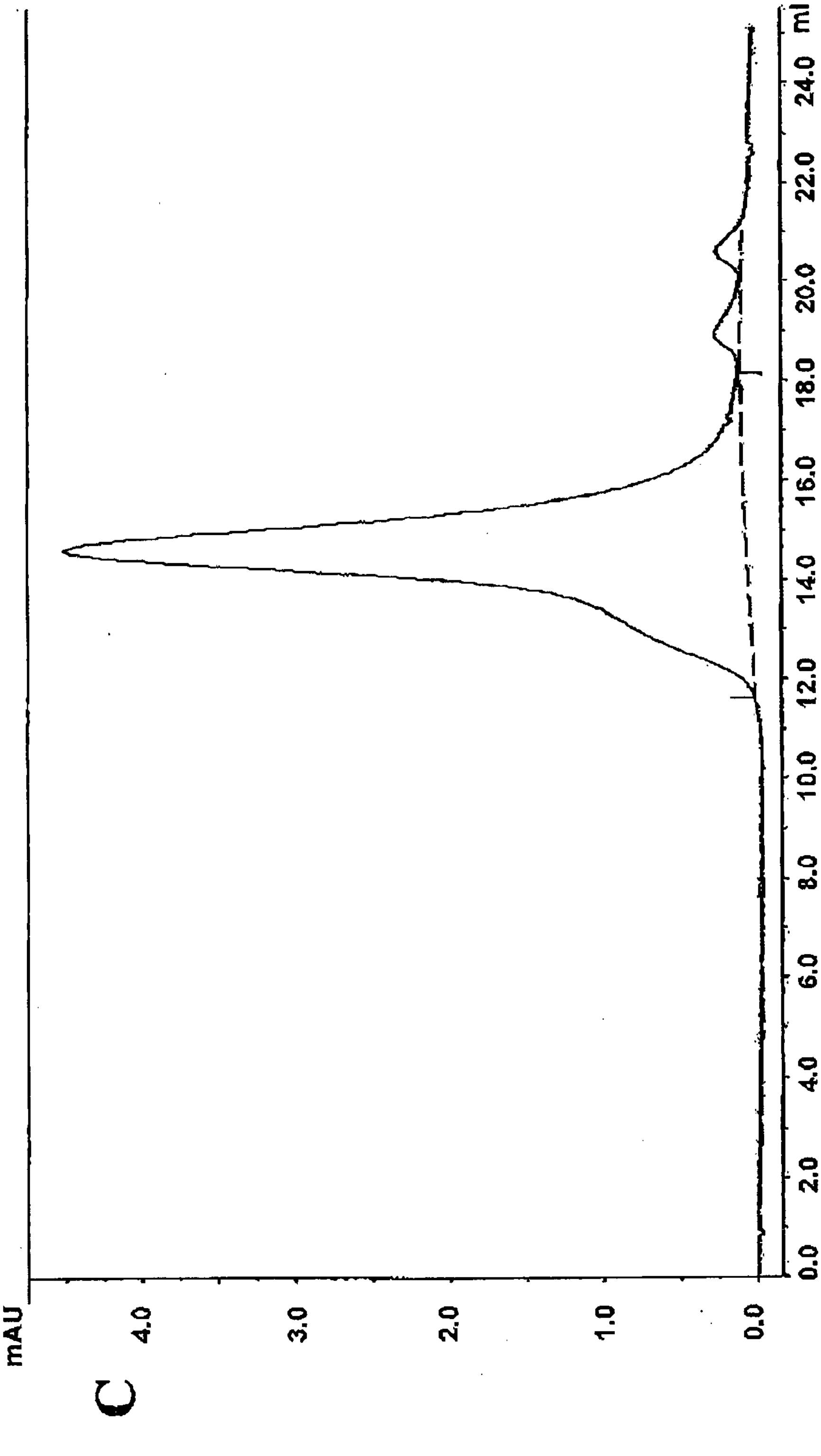
C
mAU
4.0
3.0
2.0
1.0
0.0
0.0
2.0
4.0
6.0
8.0
10.0
12.0
14.0
16.0
18.0
20.0
22.0
24.0
ml

Figure 3A

D

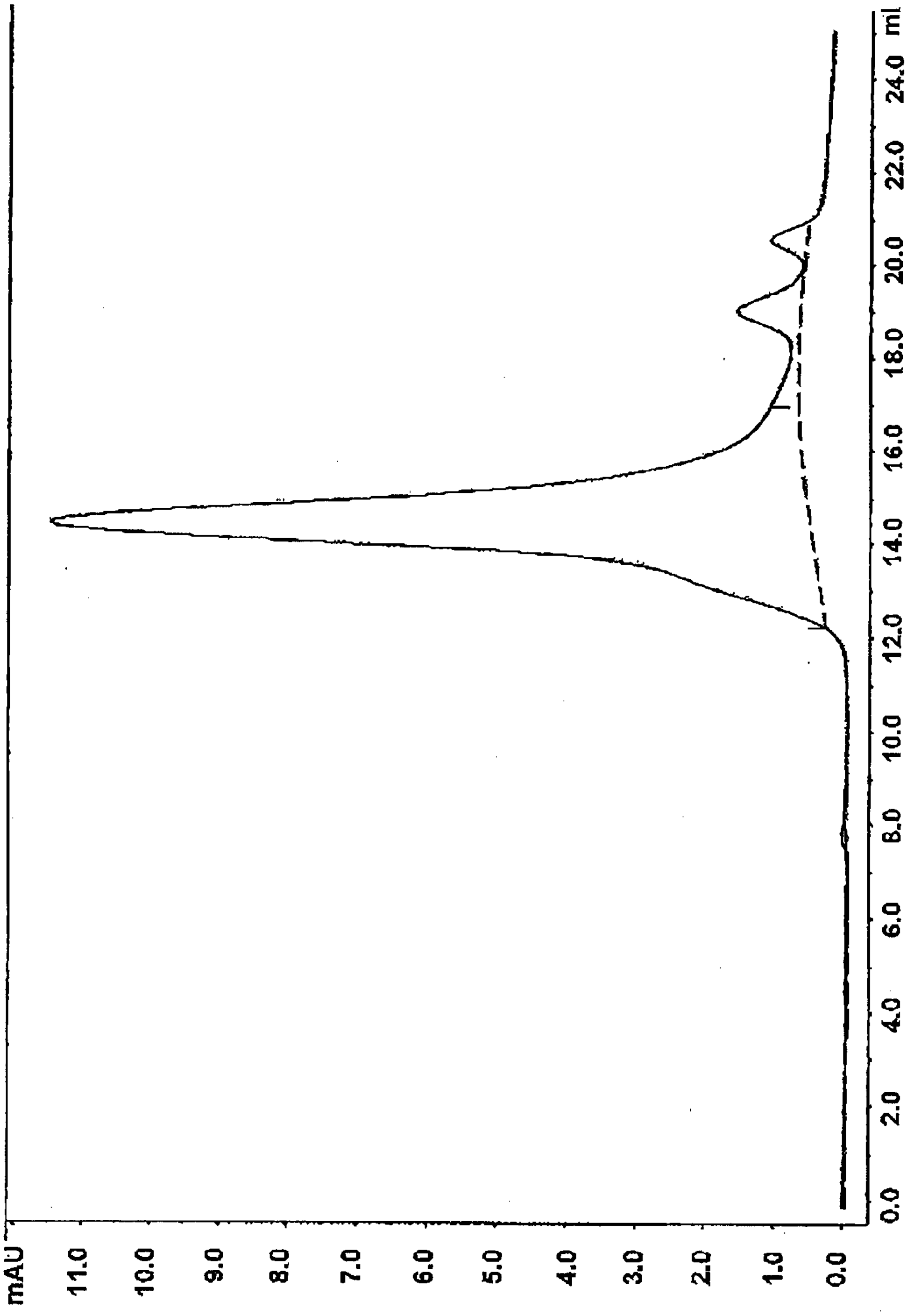
mAU
11.0
10.0
9.0
8.0
7.0
6.0
5.0
4.0
3.0
2.0
1.0
0.0
0.0
2.0
4.0
6.0
8.0
10.0
12.0
14.0
16.0
18.0
20.0
22.0
24.0
ml

Figure 3A

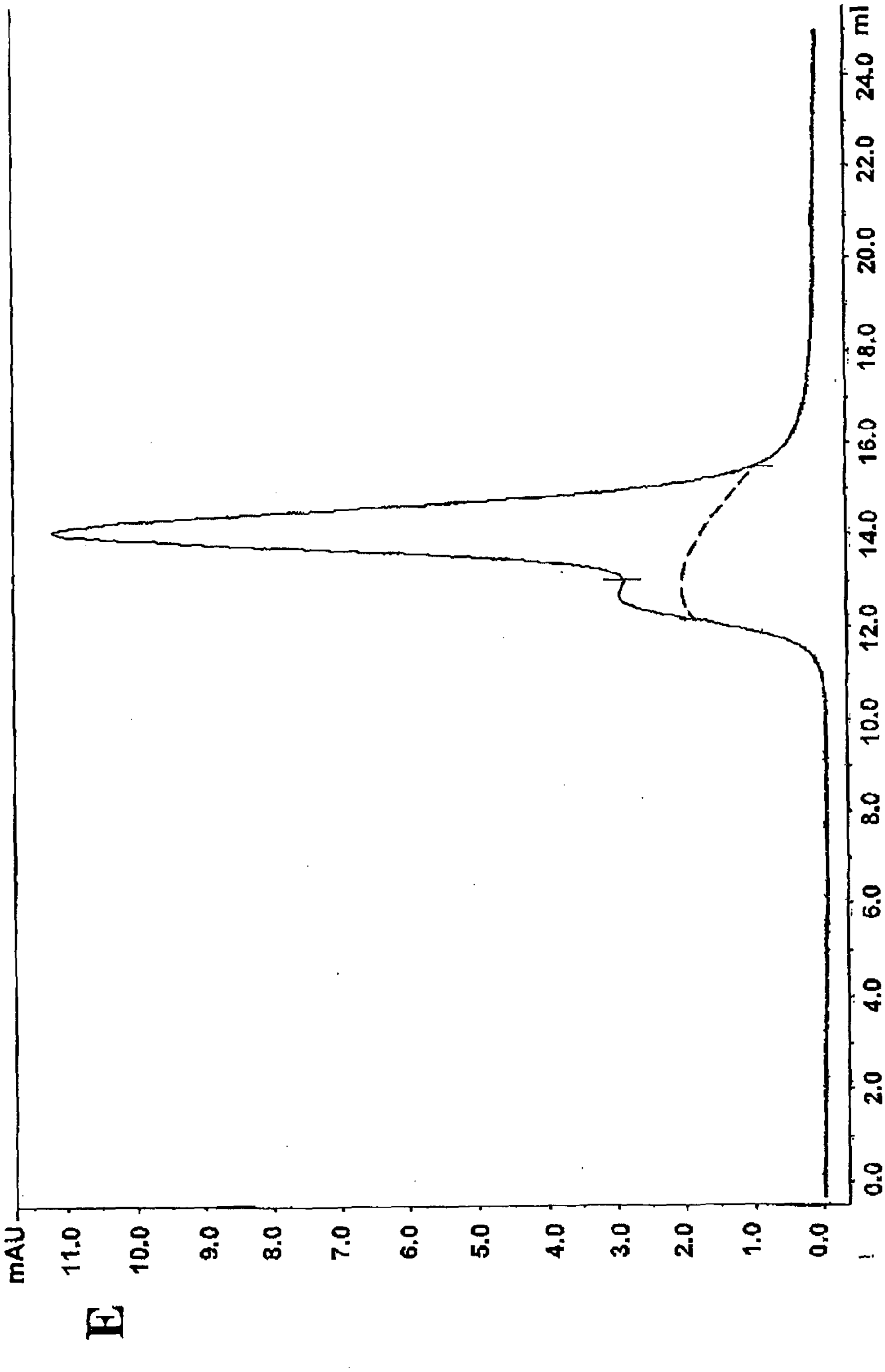
E
mAU
11.0
10.0
9.0
8.0
7.0
6.0
5.0
4.0
3.0
2.0
1.0
0.0
0.0
2.0
4.0
6.0
8.0
10.0
12.0
14.0
16.0
18.0
20.0
22.0
24.0
ml

Figure 3A

F

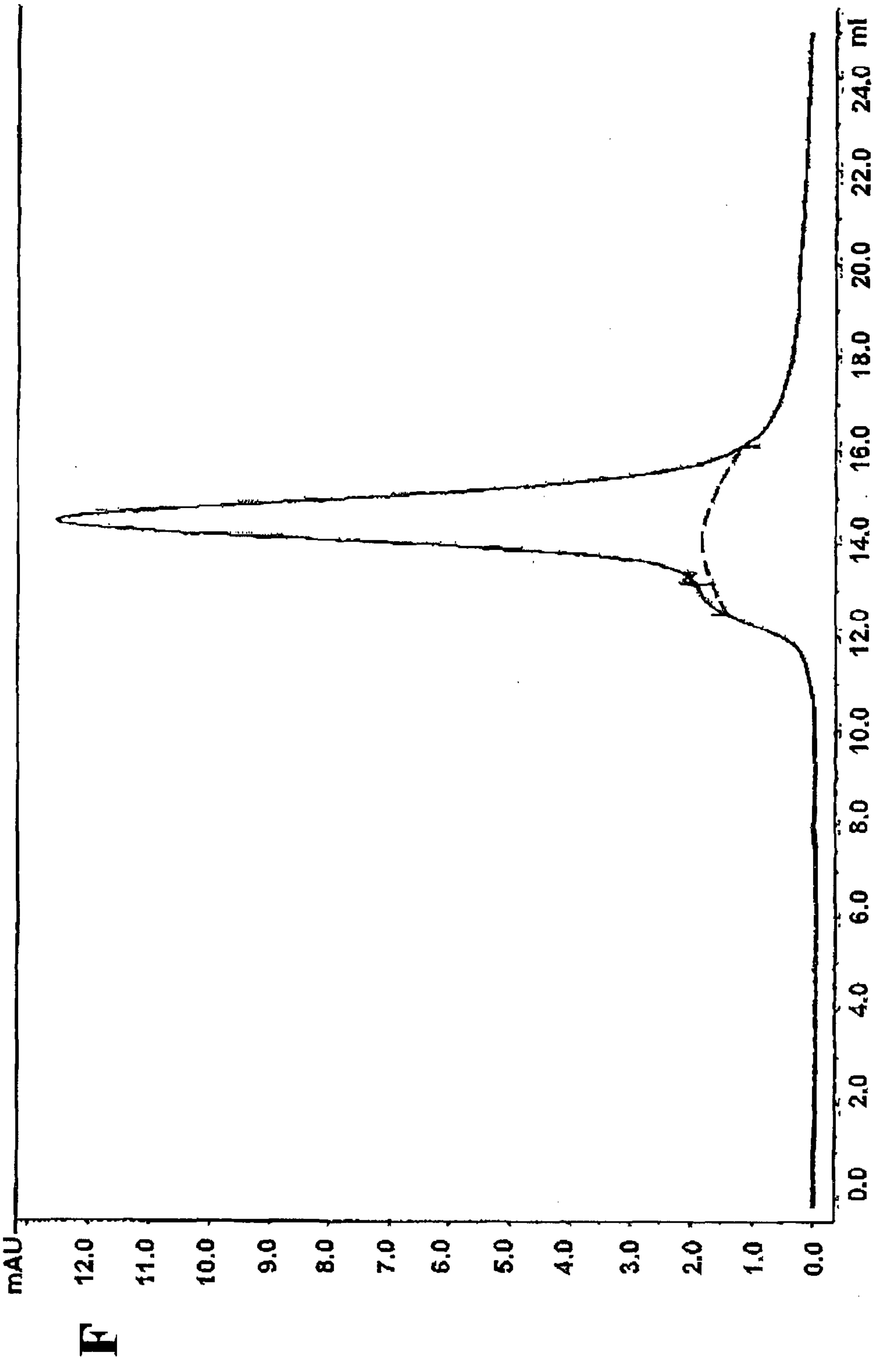
mAU
12.0
11.0
10.0
9.0
8.0
7.0
6.0
5.0
4.0
3.0
2.0
1.0
0.0
0.0
2.0
4.0
6.0
8.0
10.0
12.0
14.0
16.0
18.0
20.0
22.0
24.0
ml

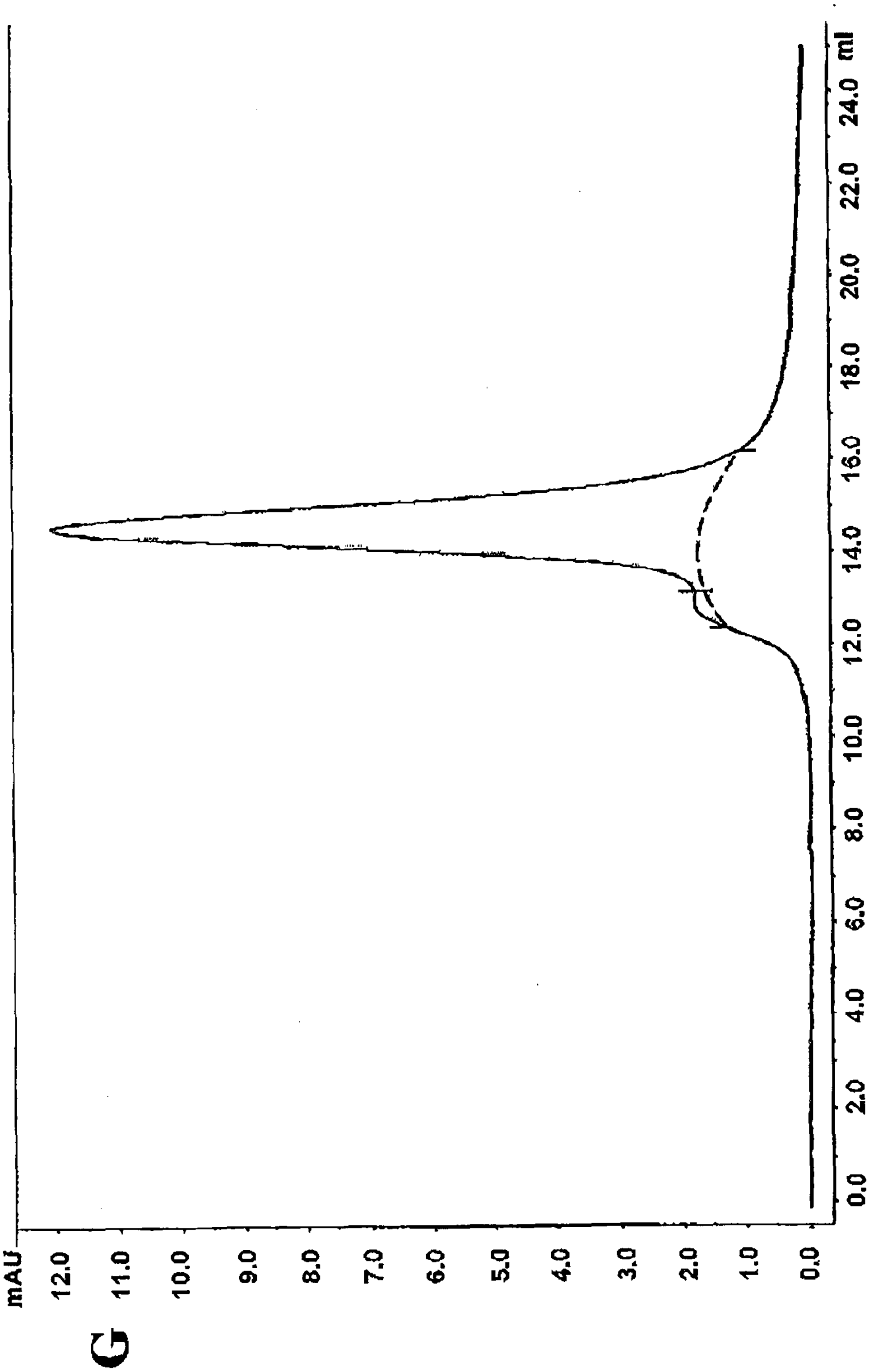
G
mAU
12.0
11.0
10.0
9.0
8.0
7.0
6.0
5.0
4.0
3.0
2.0
1.0
0.0
0.0
2.0
4.0
6.0
8.0
10.0
12.0
14.0
16.0
18.0
20.0
22.0
24.0
ml

Figure 3A

Figure 3A

H

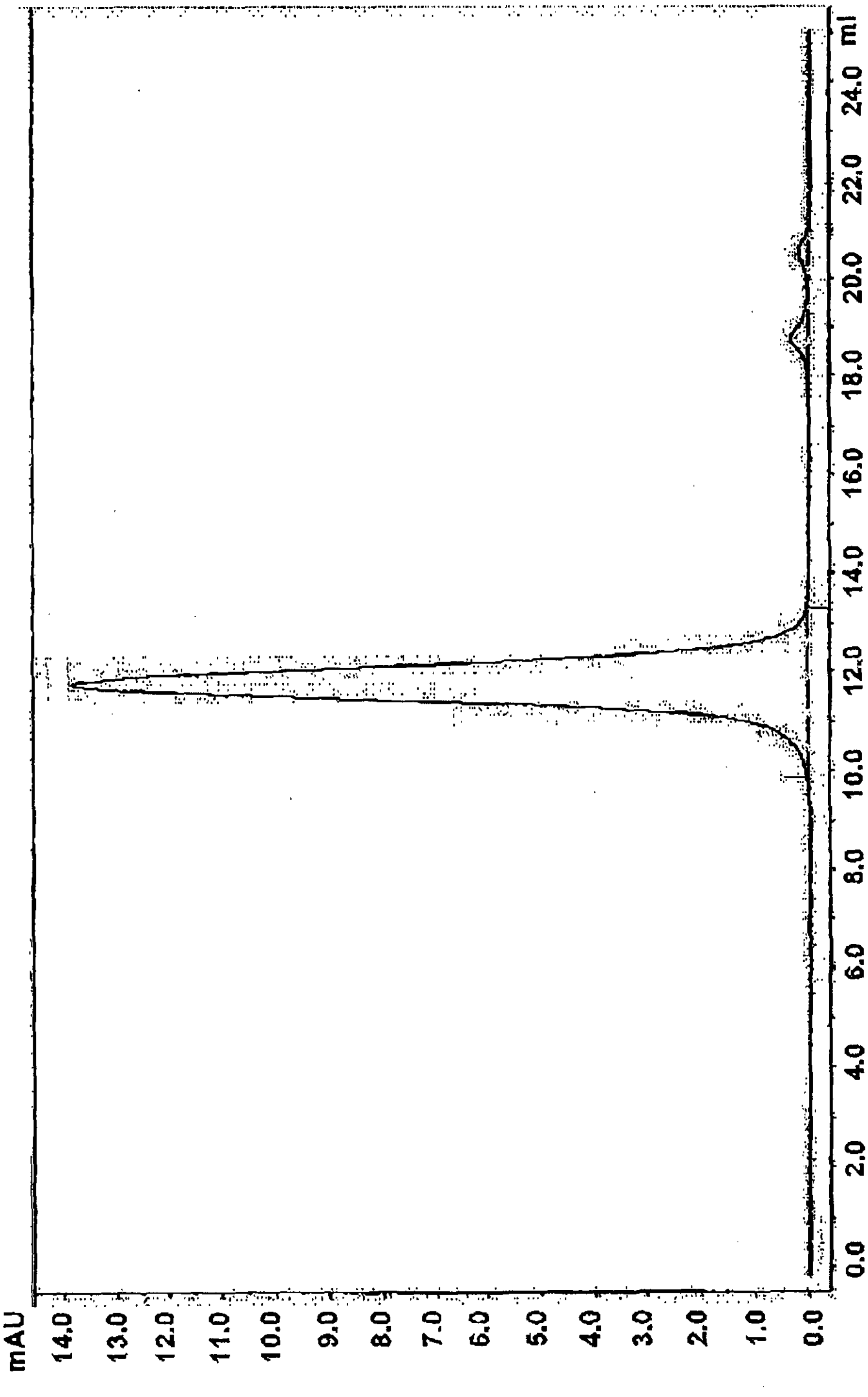
mAU
14.0
13.0
12.0
11.0
10.0
9.0
8.0
7.0
6.0
5.0
4.0
3.0
2.0
1.0
0.0
0.0
2.0
4.0
6.0
8.0
10.0
12.0
14.0
16.0
18.0
20.0
22.0
24.0
ml

Figure 3A

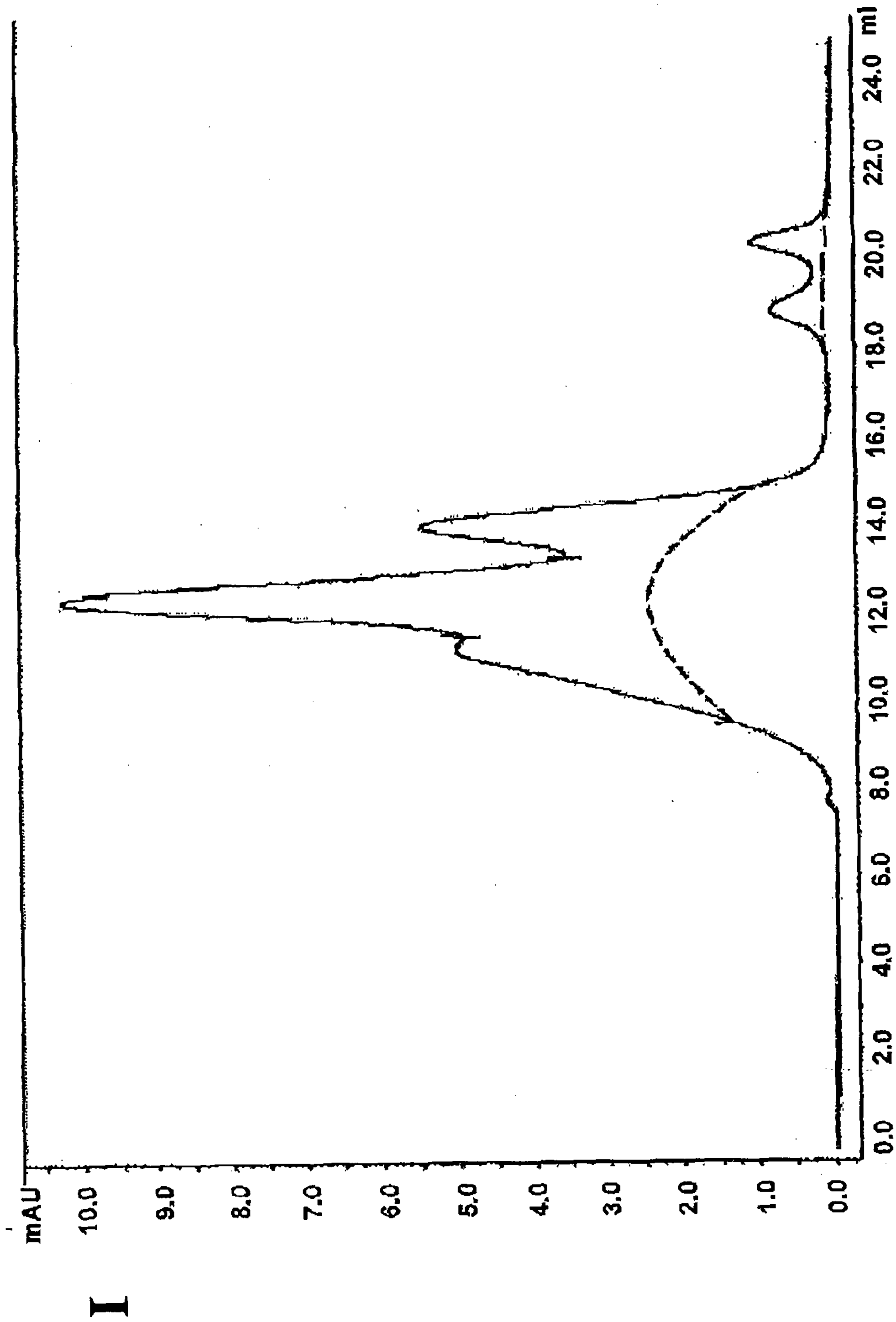
mAU
10.0
9.0
8.0
7.0
6.0
5.0
4.0
3.0
2.0
1.0
0.0
0.0
2.0
4.0
6.0
8.0
10.0
12.0
14.0
16.0
18.0
20.0
22.0
24.0
ml
I

Figure 3A

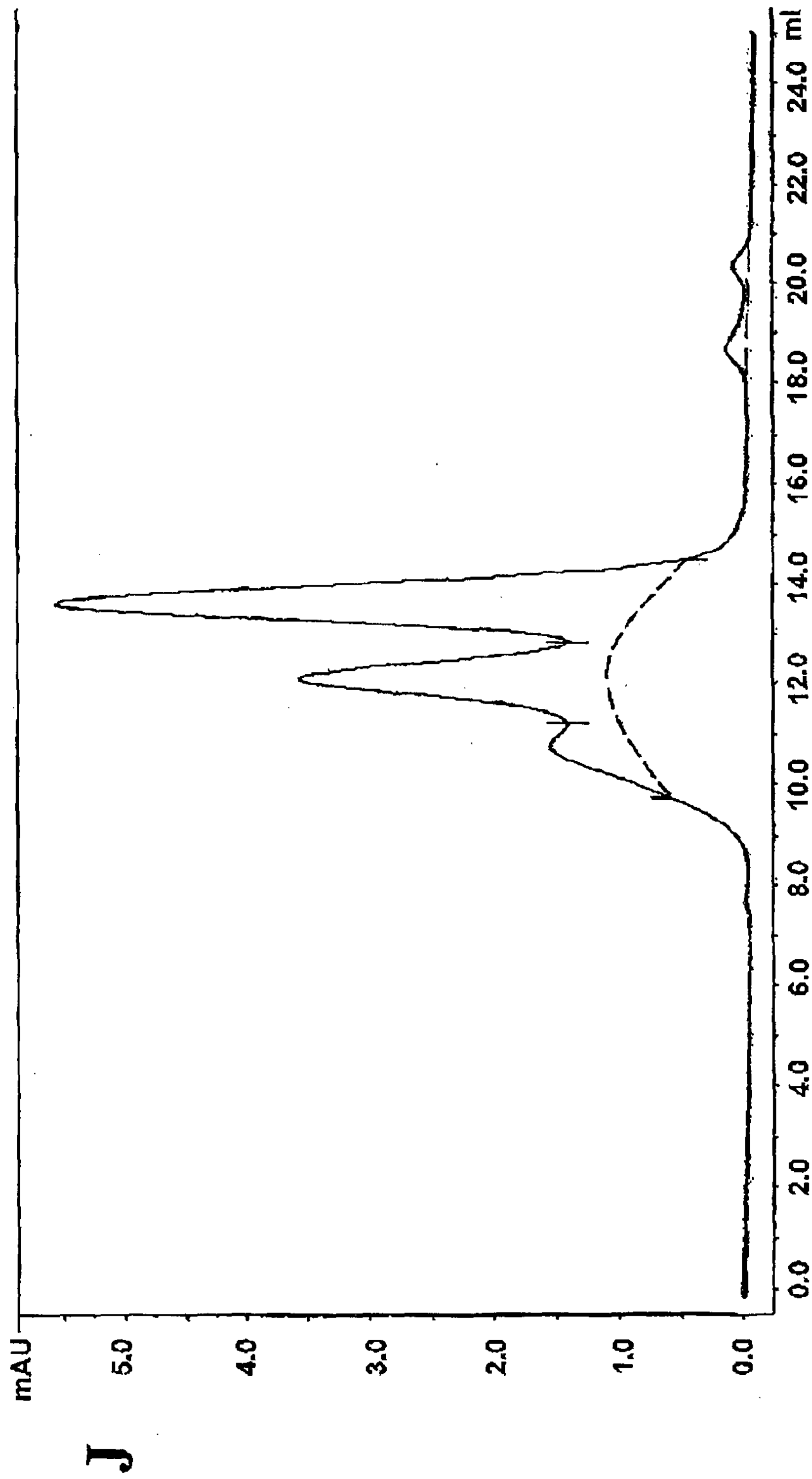
J
mAU
5.0
4.0
3.0
2.0
1.0
0.0
0.0
2.0
4.0
6.0
8.0
10.0
12.0
14.0
16.0
18.0
20.0
22.0
24.0
ml

# Figure 3B

| Antibody/BsAb | Tumor targeting ScFv intra-domain linker repeats | HT-29 cell binding (Her2/*neu*$^+$) | | Jurkat Binding (CD3$^+$) | | Yield |
|---|---|---|---|---|---|---|
| | | 10 μ/ml | 1 μ/ml | 10 μ/ml | 1 μ/ml | |
| Anti-CD3 mAb | N/A | N/A | N/A | 89.87% | 87.61% | N/A |
| Anti-Her2/*neu* mAb | N/A | 99.54% | 99.59% | N/A | N/A | N/A |
| Her2/*neu* targeting Ka LCBTA-1 | 3 | 98.16% | 84.15% | 76.33% | 50% | ≤0.7 mg/L |
| Her2/*neu* targeting Ka LCBTA-1-A | 2 | 97.77% | 95.6% | 82.97% | 67.57% | ≥1.5 mg/L |
| Her2/*neu* targeting Ka LCBTA-1-B | 4 | 98.64% | 97.91% | 77.51% | 61.43% | ≥1.5 mg/L |

Figure 3B(Cont.)

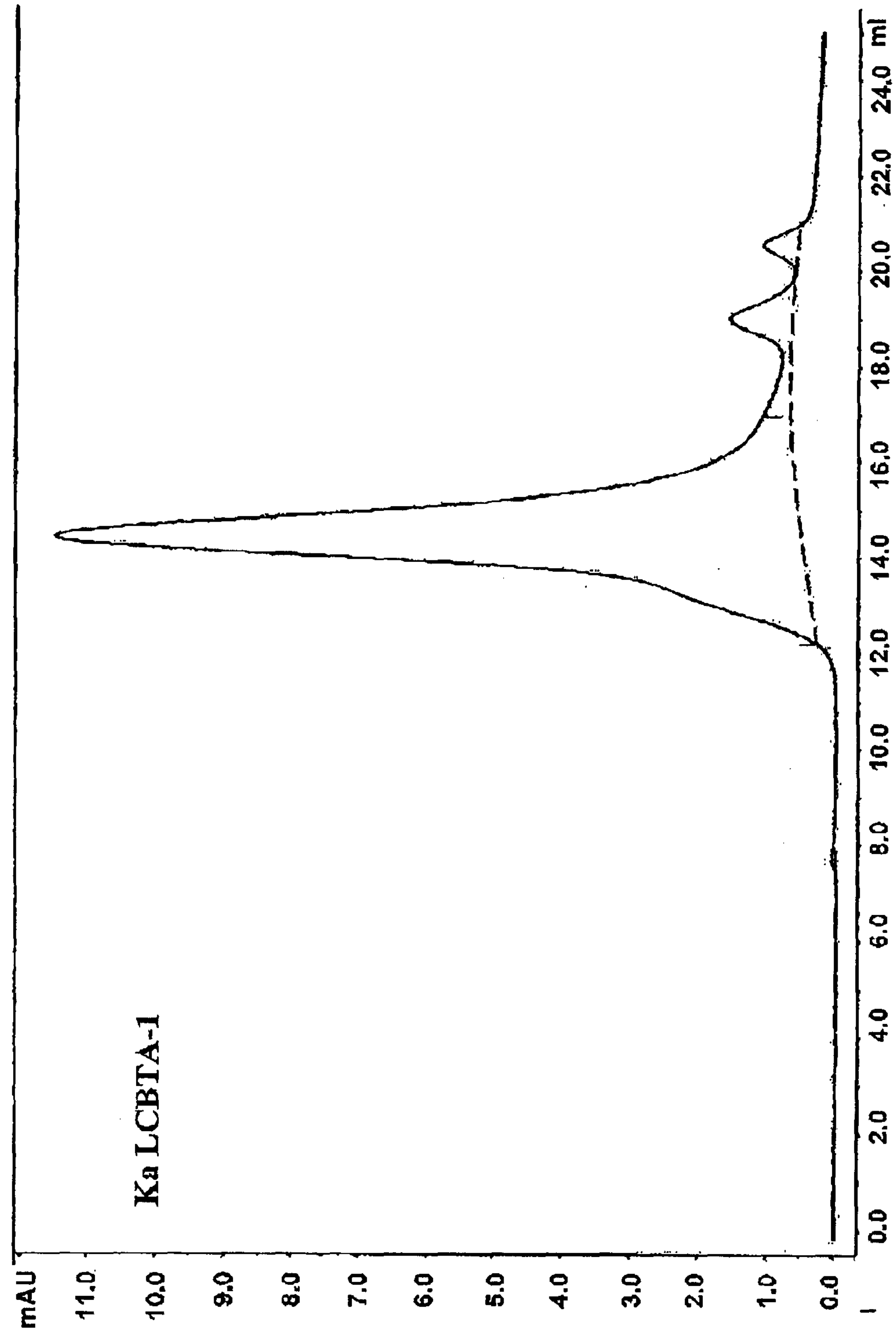

# Figure 3B (Cont.)

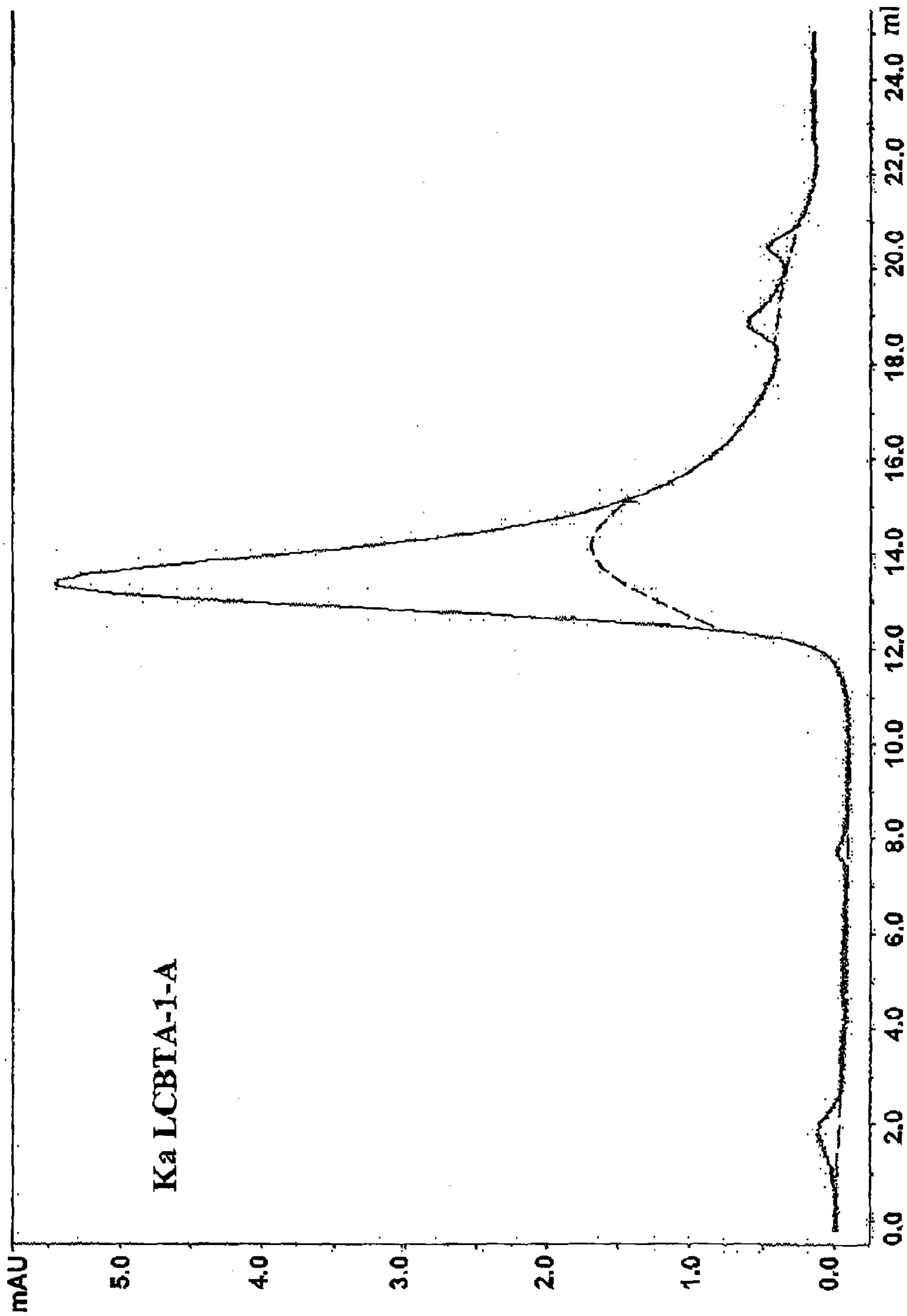

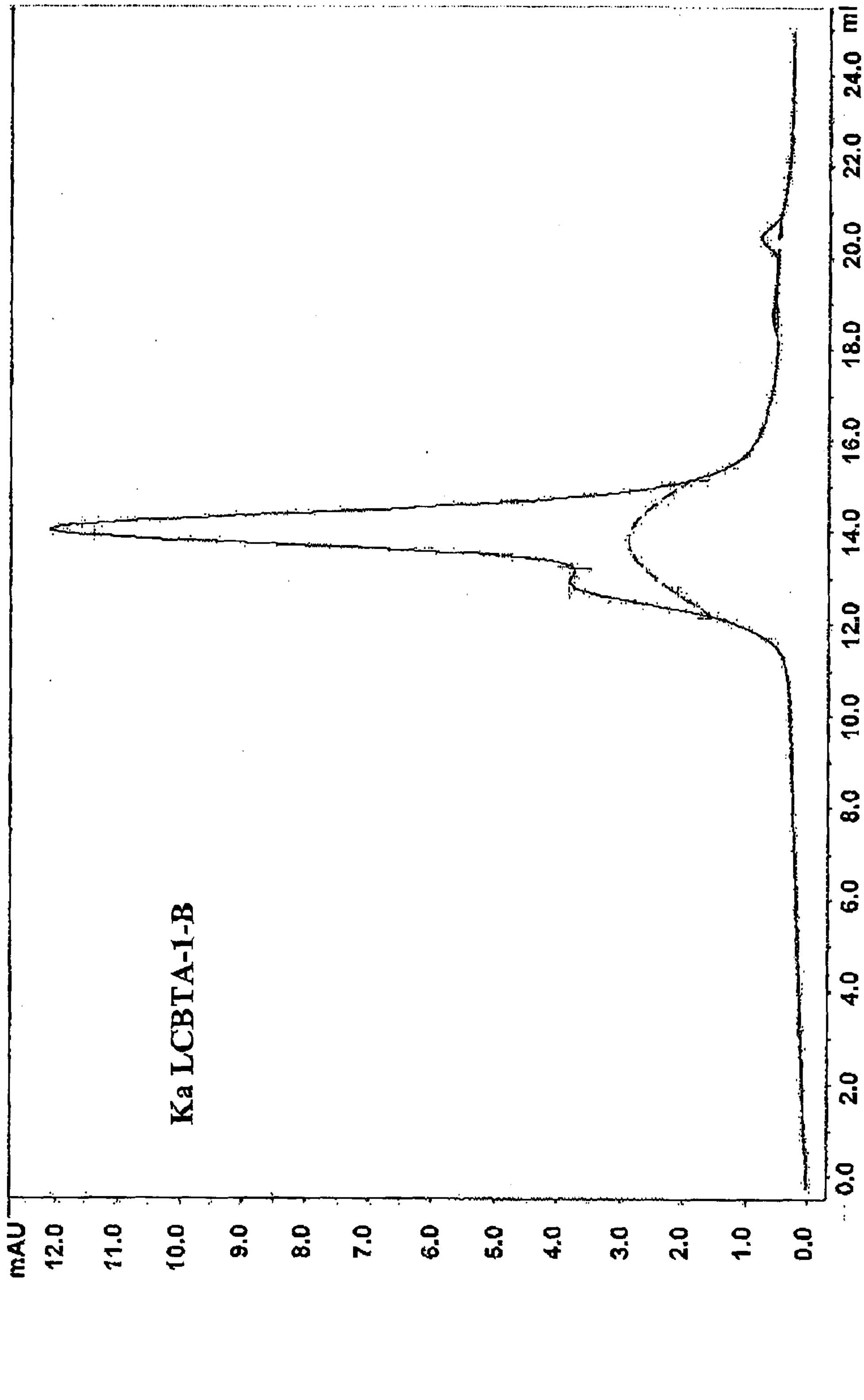


Figure 3B (Cont.)

Figure 4A

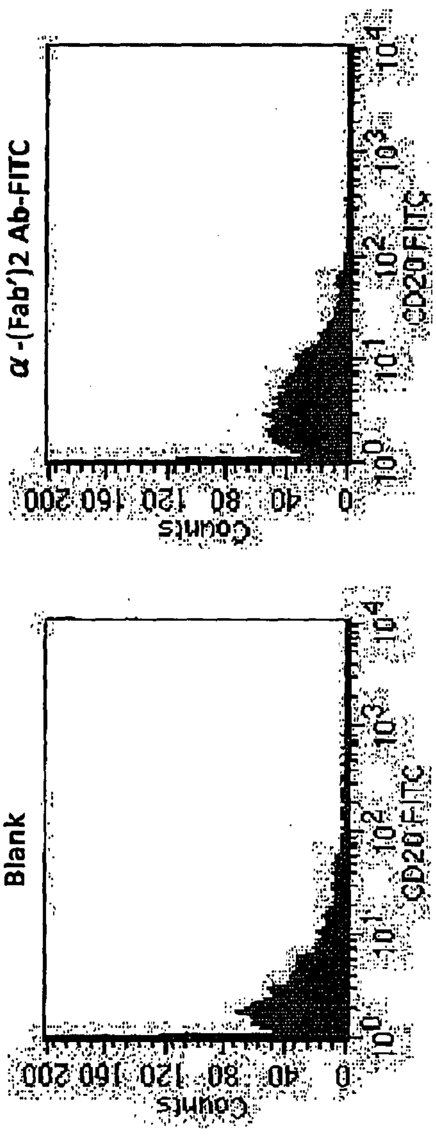
α -(Fab')2 Ab-FITC
Blank
Counts
0
40
80
120
160
200
CD20 FITC
$10^0$
$10^1$
$10^2$
$10^3$
$10^4$

Figure 4A (Continued)

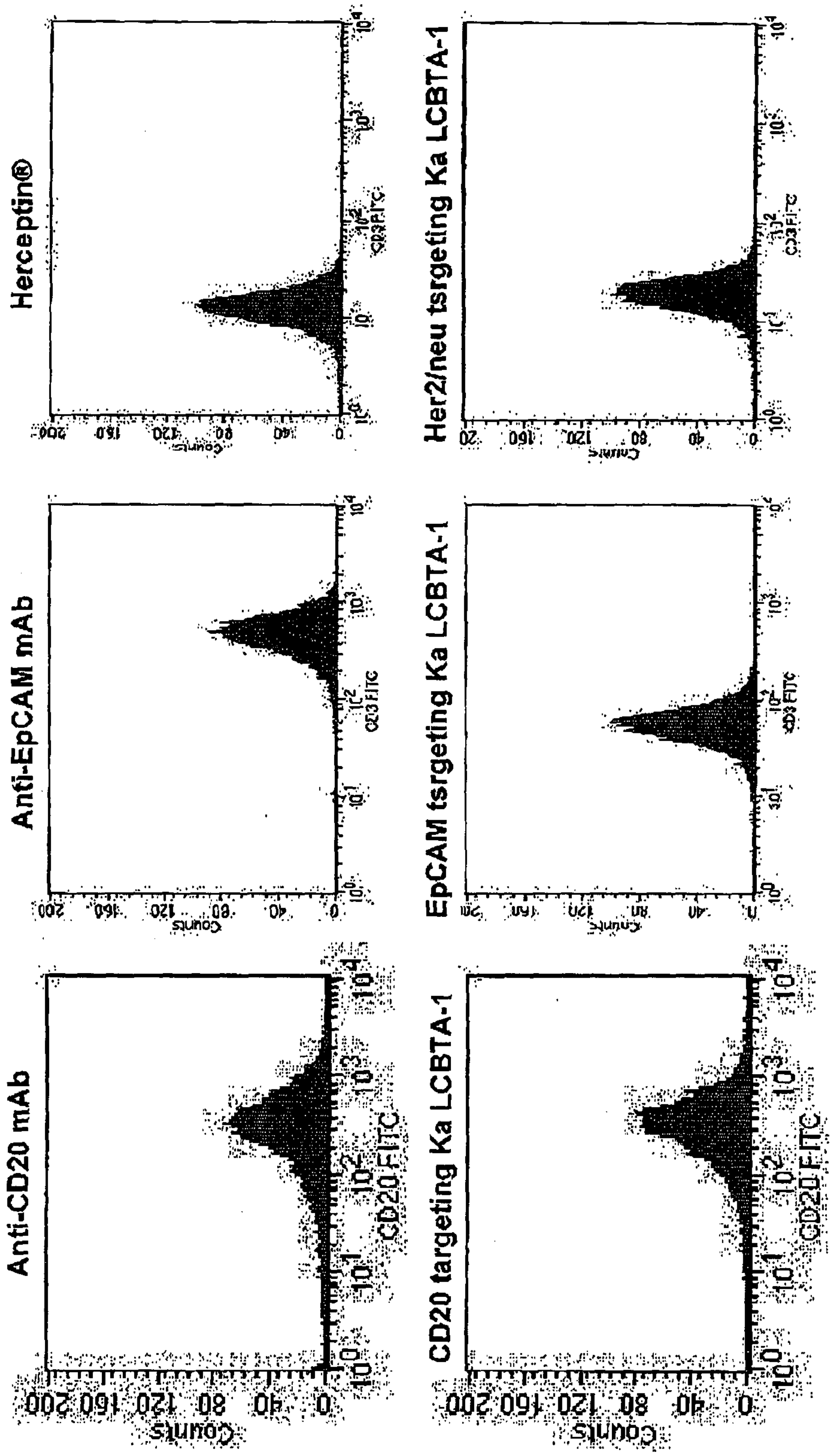

Figure 4B

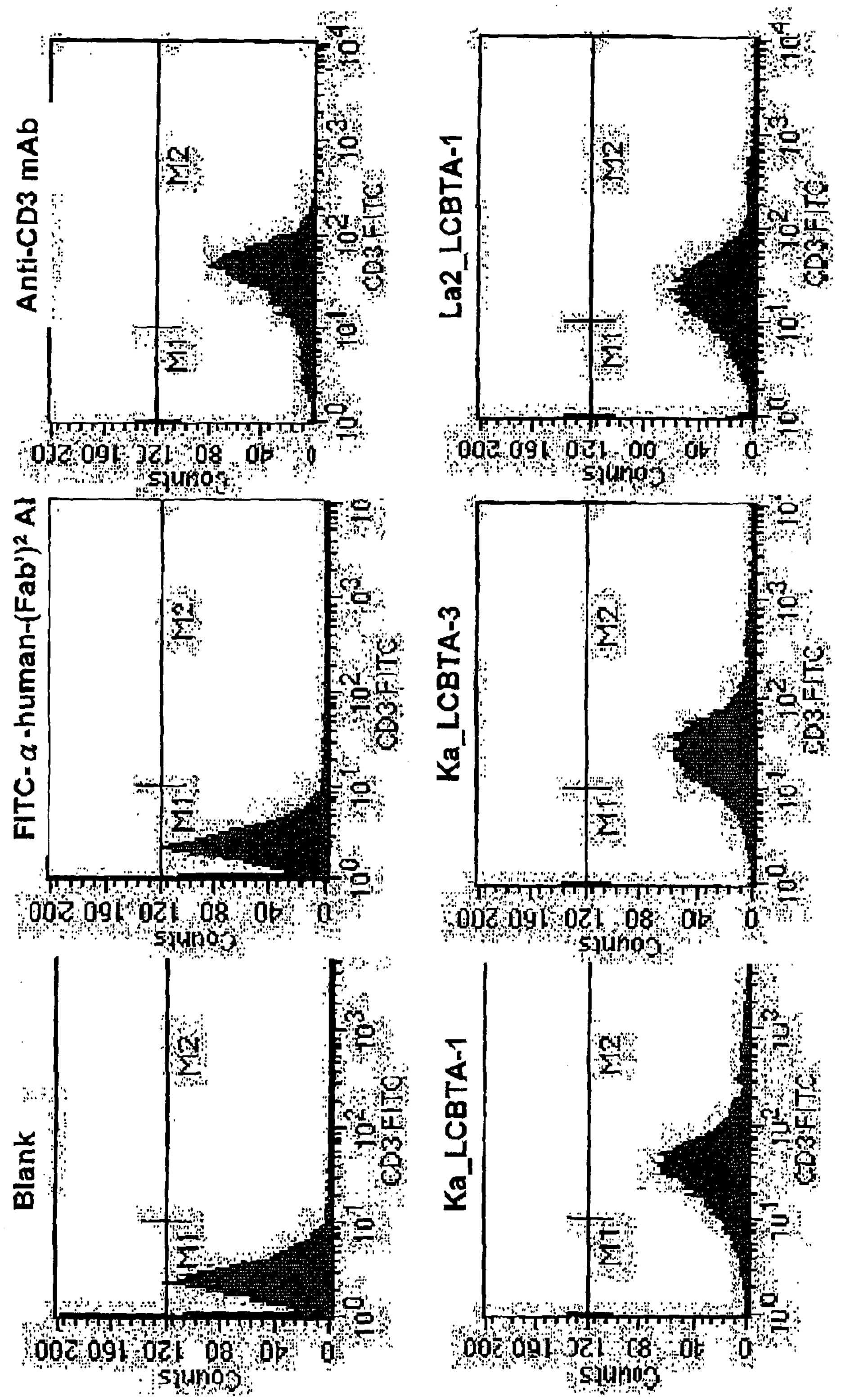
Blank
FITC-α-human-(Fab')² Ab
Anti-CD3 mAb
Ka_LCBTA-1
Ka_LCBTA-3
La2_LCBTA-1
M1
M2
Counts
CD3 FITC

Figure 4B (Continued)

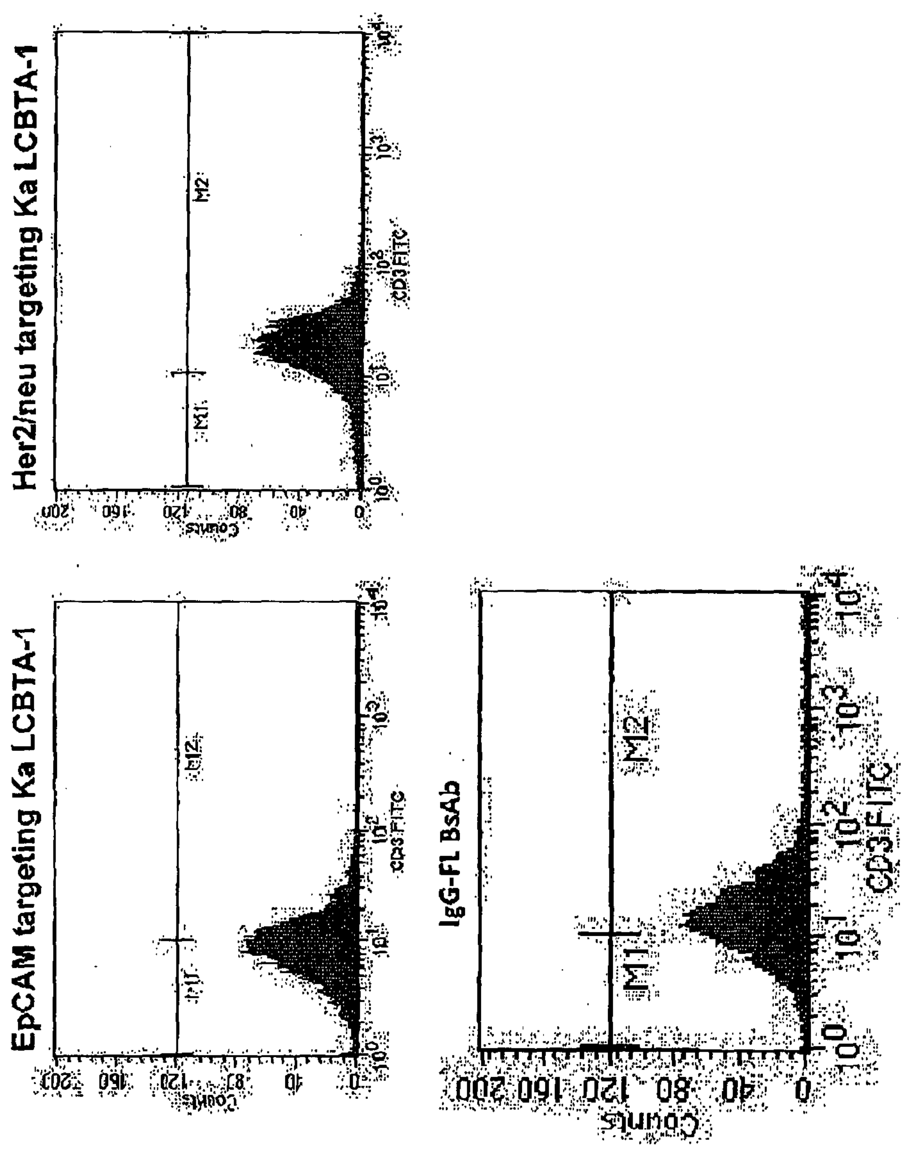

# Figure 5

A

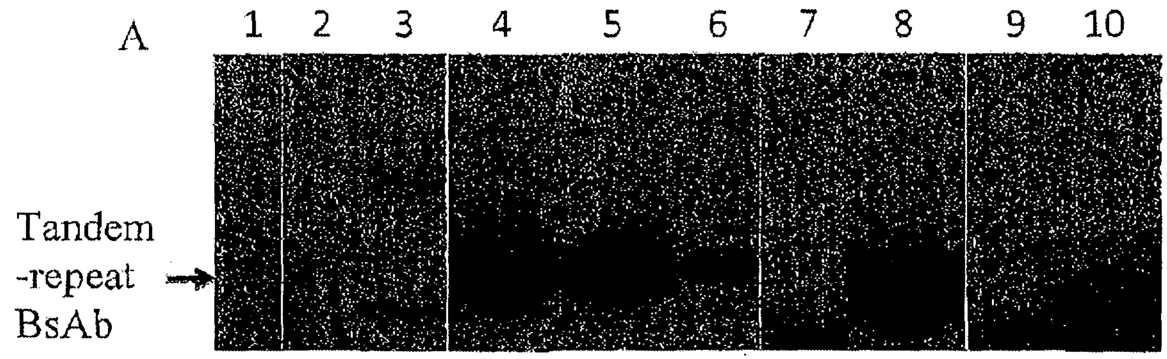


| | Yield* (µg/ml) | | Yield* (µg/ml) |
|---|---|---|---|
| 1. Tandem-repeat-1 | ≤0.05 | 7. La5 LCBTA-3 | N/A |
| 2. Tandem-repeat-2 | ≤0.05 | 8. Ka LCBTA-1 | ≥1 |
| 3. CH-1 bridged | ≤0.05 | 9. Ka LCBTA-3 | ≥0.7 |
| 4. La2 LCBTA-1 | ≥1 | 10. IgG-FL BsAb | ≥1 |
| 5. La2 LCBTA-3 | ≥0.9 | | |
| 6. La5 LCBTA-1 | N/A | | |

Yield could vary due to variations of targeting molecules

Figure 6

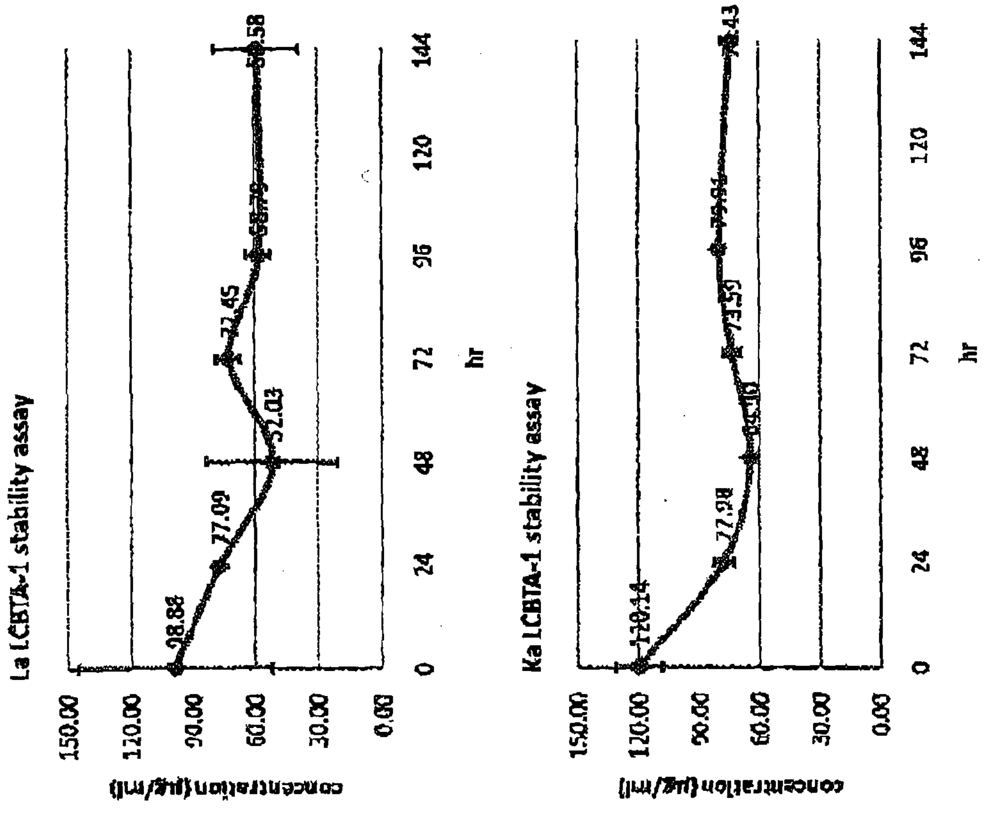
La LCBTA-1 stability assay
concentration (μg/ml)
150.00
120.00
90.00
60.00
30.00
0.00
0
24
48
72
96
120
144
hr
98.88
77.09
52.03
72.45
58.58
Ka LCBTA-1 stability assay
concentration (μg/ml)
150.00
120.00
90.00
60.00
30.00
0.00
0
24
48
72
96
120
144
hr
120.14
77.98
73.59
78.43

Figure 7

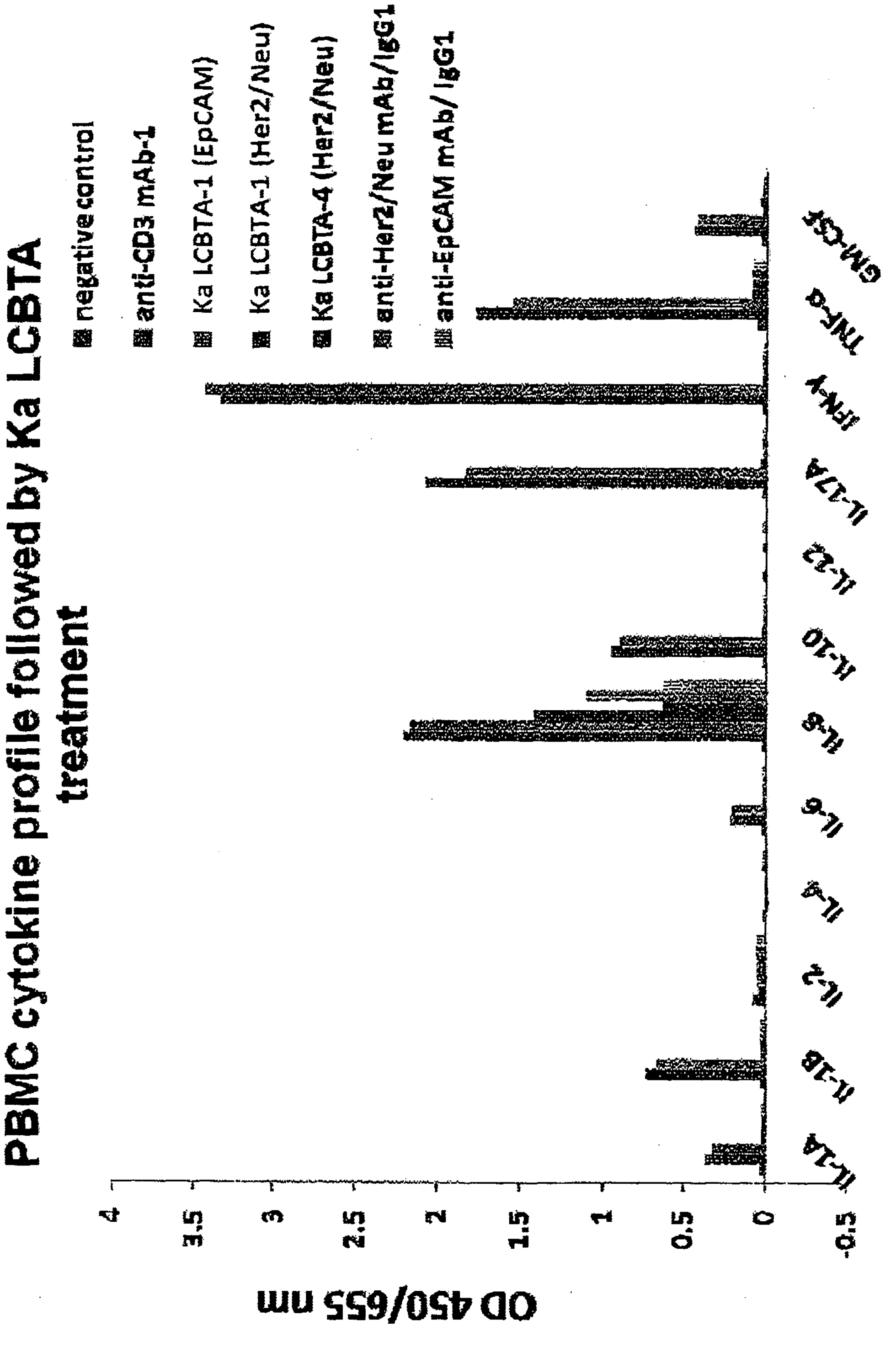
PBMC cytokine profile followed by Ka LCBTA treatment
negative control
anti-CD3 mAb-1
Ka LCBTA-1 (EpCAM)
Ka LCBTA-1 (Her2/Neu)
Ka LCBTA-4 (Her2/Neu)
anti-Her2/Neu mAb/IgG1
anti-EpCAM mAb/ IgG1
OD 450/655 nm
4
3.5
3
2.5
2
1.5
1
0.5
0
-0.5
IL-1A
IL-1B
IL-2
IL-4
IL-6
IL-8
IL-10
IL-12
IL-17A
IFN-γ
TNF-α
GM-CSF

Figure 8A

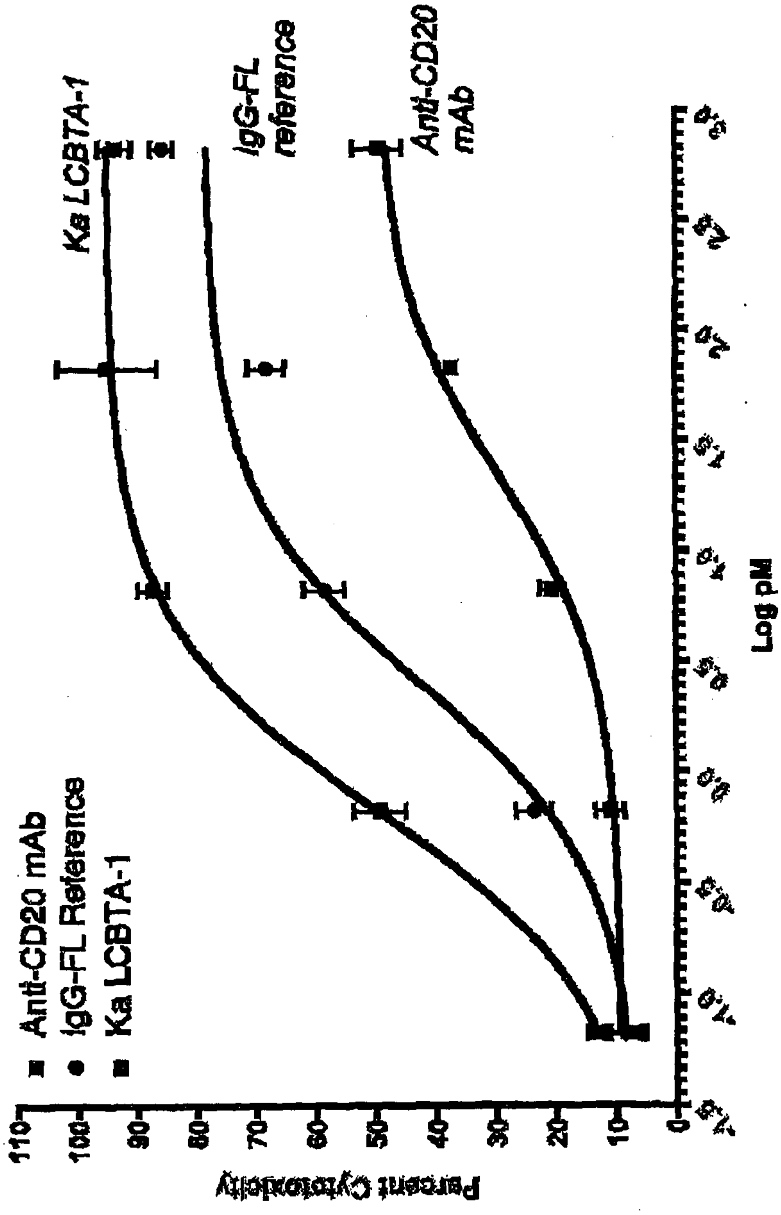

Anti-CD20 mAb
IgG-FL Reference
Ka LCBTA-1
Ka LCBTA-1
IgG-FL reference
Anti-CD20 mAb
Percent Cytotoxicity
110
100
90
80
70
60
50
40
30
20
10
0
-1.5
-1.0
-0.5
0.0
0.5
1.0
1.5
2.0
2.5
3.0
Log pM

Figure 8B

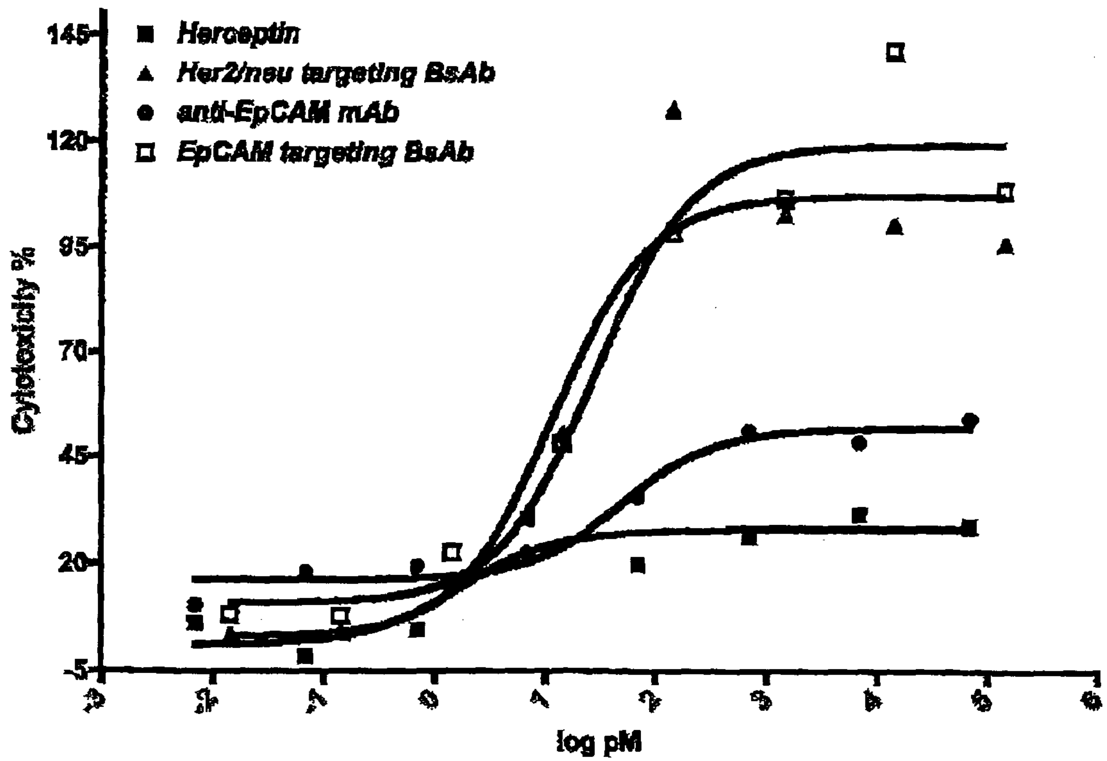
Herceptin
Her2/neu targeting BsAb
anti-EpCAM mAb
EpCAM targeting BsAb
145
120
95
70
45
20
-5
Cytotoxicity %
log pM

Figure 8C

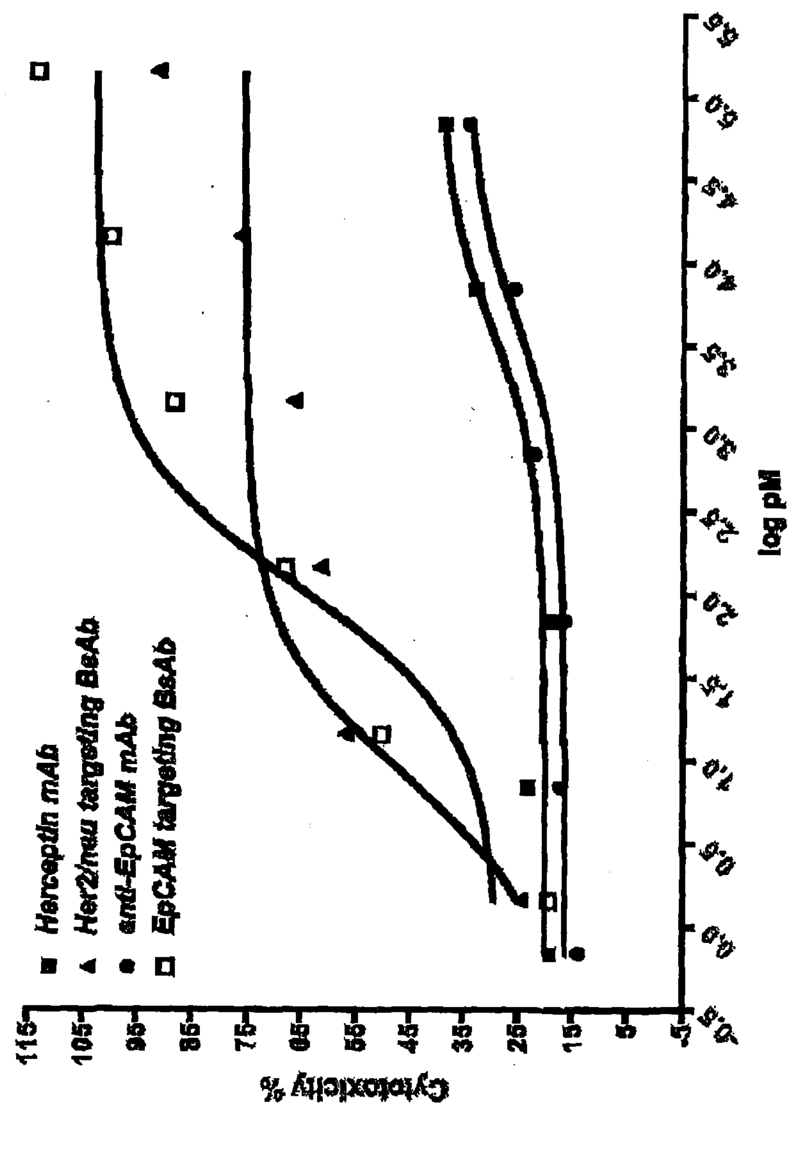
Herceptin mAb
Her2/neu targeting BsAb
anti-EpCAM mAb
EpCAM targeting BsAb
Cytotoxicity %
115
105
95
85
75
65
55
45
35
25
15
5
-5
-0.5
0.0
0.5
1.0
1.5
2.0
2.5
3.0
3.5
4.0
4.5
5.0
5.5
log pM

Figure 9A

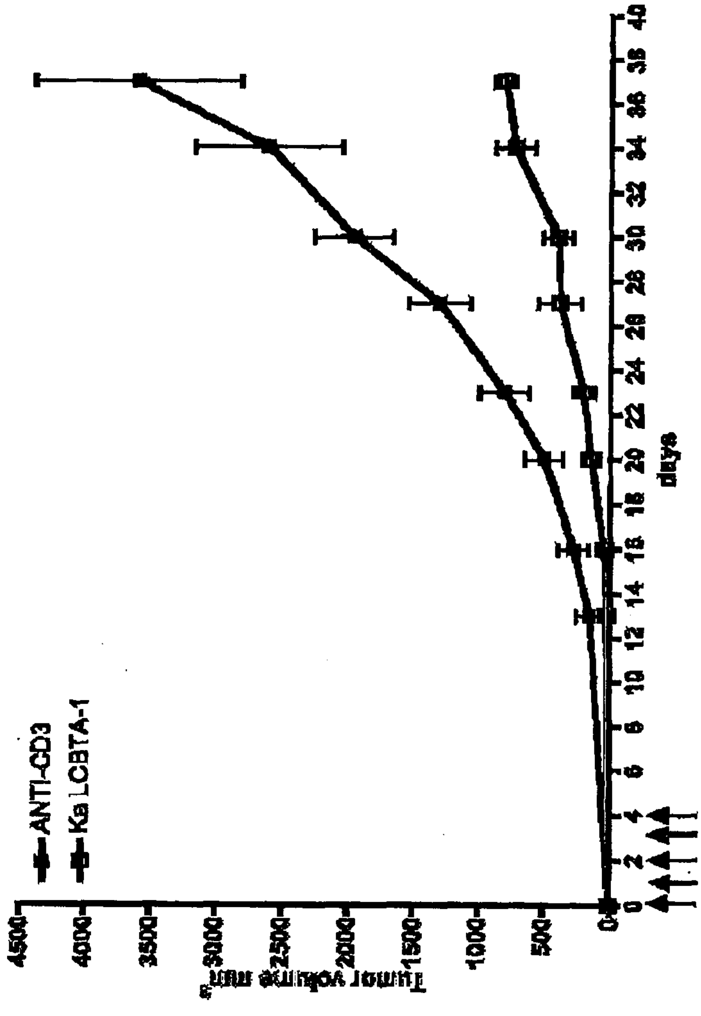
ANTI-CD3
Ka LCBTA-1
Tumor volume mm3
days
0
500
1000
1500
2000
2500
3000
3500
4000
4500
0 2 4 6 8 10 12 14 16 18 20 22 24 26 28 30 32 34 36 38 40

Figure 9B

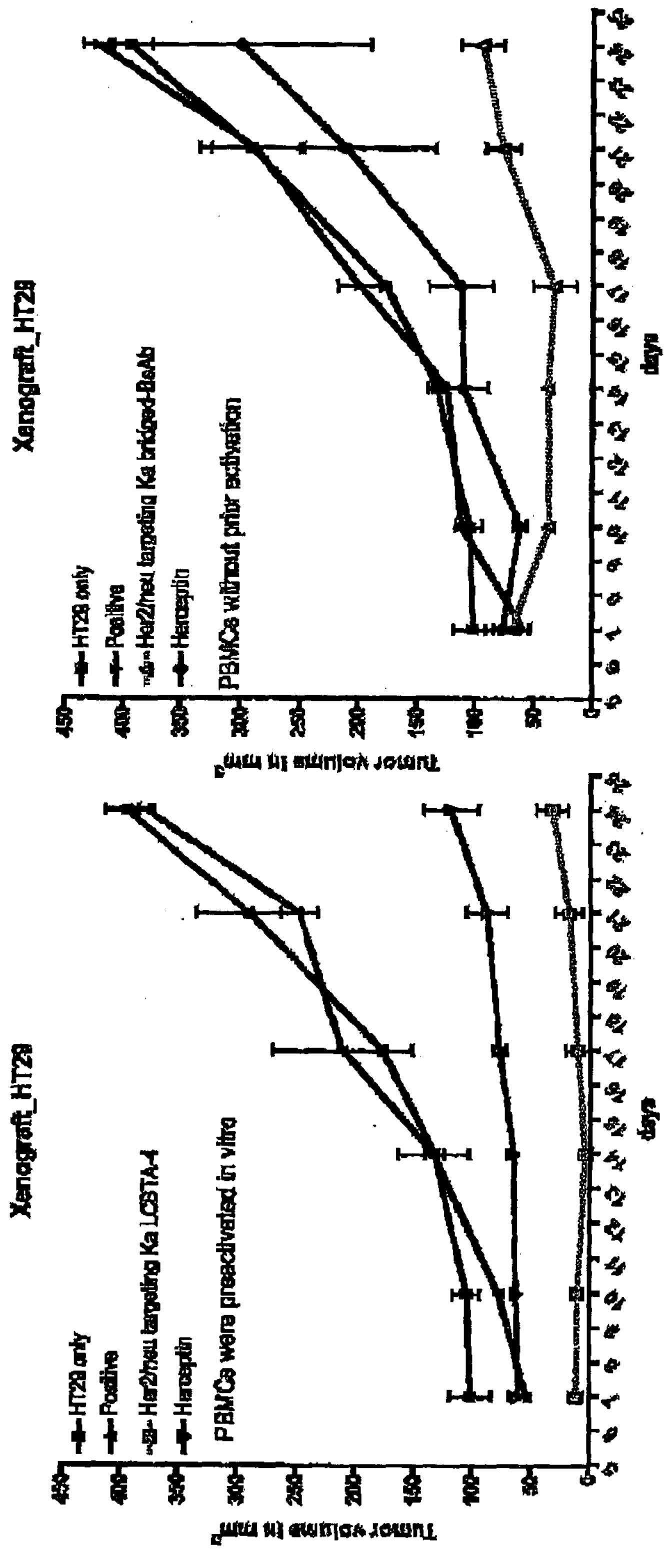
Xenograft_HT29
HT29 only
Positive
Her2/neu targeting Ka LCSTA-4
Herceptin
PBMCs were preactivated in vitro
Tumor volume in mm3
days
Xenograft_HT29
HT29 only
Positive
Her2/neu targeting Ka bridged-BsAb
Herceptin
PBMCs without prior activation
Tumor volume in mm3
days

Figure 9C

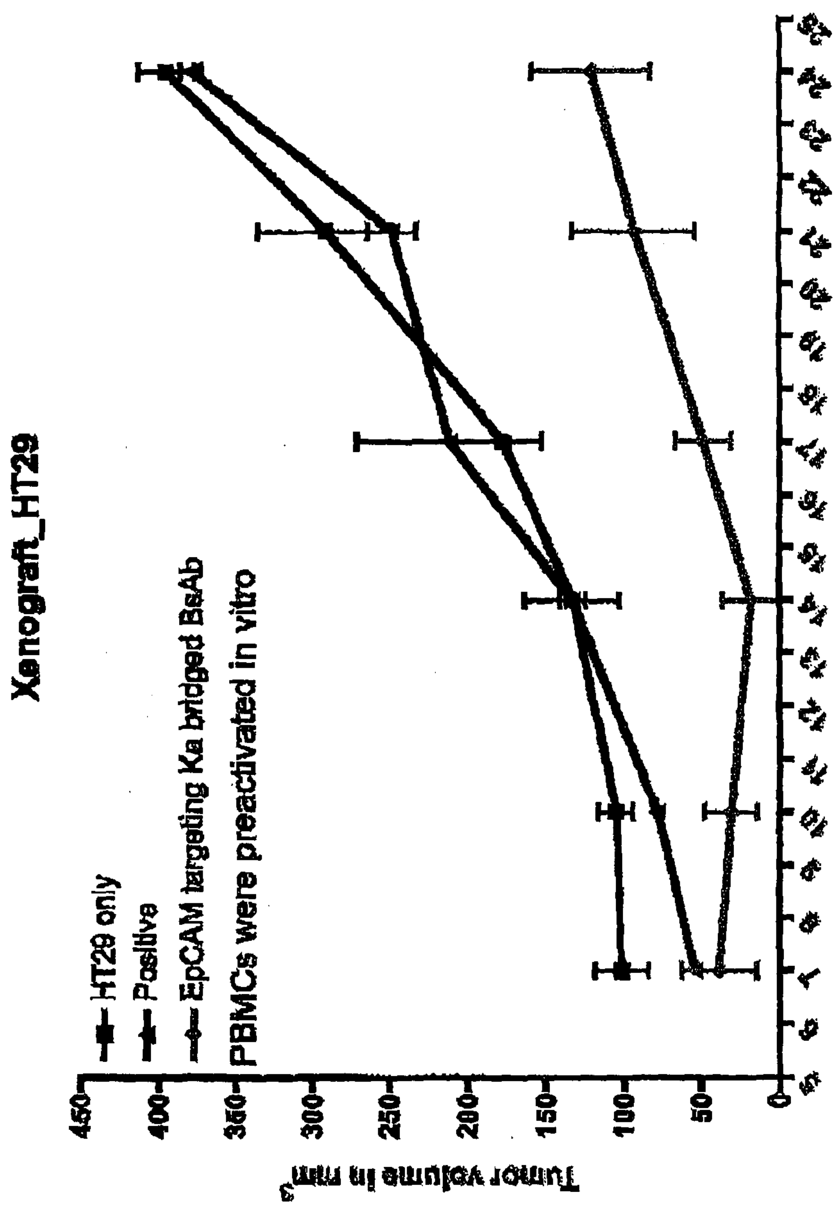
Xenograft_HT29
HT29 only
Positive
EpCAM targeting Ka bridged BsAb
PBMCs were preactivated in vitro
Tumor volume in mm3
450
400
350
300
250
200
150
100
50
0
days

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Mol. Imm.,* 1996, vol. 33 (2), 211-219
- *Landes Bioscience,* 2009, vol. 1 (6), 529-547
- *International Journal of Molecular Medicine,* 2010, vol. 25 (2), 209-215
- *Hybridoma.,* 1983, vol. 2, 17
- *Am J Clin Pathol,* 2003, vol. 120 (1), S53
- *J. Immunol.,* 1992, vol. 148 (2), 590
- Cancer Res. Biopharmaceutical society, 08 August 2000, vol. 60, 433